(19) Europäisches Patentamt
European Patent Office
Office européen des brevets

(11) **EP 2 197 830 B1**

(12) **EUROPÄISCHE PATENTSCHRIFT**

(45) Veröffentlichungstag und Bekanntmachung des Hinweises auf die Patenterteilung:
**02.05.2018 Patentblatt 2018/18**

(21) Anmeldenummer: **08787270.1**

(22) Anmeldetag: **15.08.2008**

(51) Int Cl.:
*C07C 209/16* (2006.01)    *C07C 211/09* (2006.01)
*C07C 211/10* (2006.01)    *C07C 211/11* (2006.01)
*C07C 213/02* (2006.01)    *C07C 215/08* (2006.01)
*C04B 24/12* (2006.01)    *C04B 28/02* (2006.01)
*C07D 241/04* (2006.01)    *C07D 295/02* (2006.01)

(86) Internationale Anmeldenummer:
**PCT/EP2008/060742**

(87) Internationale Veröffentlichungsnummer:
**WO 2009/027249 (05.03.2009 Gazette 2009/10)**

(54) **VERFAHREN ZUR HERSTELLUNG VON AMINEN AUS ZUCKERALKOHOLEN**

METHOD FOR PRODUCING AMINES FROM SUGAR ALCOHOLS

PROCÉDÉ POUR PRODUIRE DES AMINES À PARTIR DE POLYOLS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **29.08.2007 EP 07115219**

(43) Veröffentlichungstag der Anmeldung:
**23.06.2010 Patentblatt 2010/25**

(73) Patentinhaber: **BASF SE**
**67056 Ludwigshafen am Rhein (DE)**

(72) Erfinder:
• **ERNST, Martin**
**69115 Heidelberg (DE)**
• **HOFFER, Bram Willem**
**Fanwood, NJ 07023 (US)**
• **MELDER, Johann-Peter**
**67459 Böhl-Iggelheim (DE)**

(74) Vertreter: **BASF IP Association**
**BASF SE**
**G-FLP-C006**
**67056 Ludwigshafen (DE)**

(56) Entgegenhaltungen:
WO-A-2005/051874    US-A- 2 004 135
US-A- 2 852 570

• FISCHER A ET AL: "Amination of diols and polyols to acyclic amines" CATALYSIS TODAY, ELSEVIER, Bd. 37, 1. Januar 1997 (1997-01-01), Seiten 167-189, XP002499395 ISSN: 0920-5861 in der Anmeldung erwähnt
• KELKENBERG H: "Detergenzien auf Zuckerbasis. Neue Komponenten für Waschrohstoffe und Kosmetika" TENSIDE, SURFACTANTS, DETERGENTS, CARL HANSER VERLAG, MUNCHEN, DE, Bd. 25, Nr. 1, 1. Januar 1988 (1988-01-01), Seiten 8-13, XP009115361 ISSN: 0932-3414 in der Anmeldung erwähnt
• KARSTEN ELLER, ERHARD HENKES, ROLAND ROSSBACHER, HARTMUT HÖKE: "Ullmann's Encyclopedia of industrial Chemistry; 6th ed; A2, Amines aliphatic;" 2002, VCH , WEINHEIM , XP002525107 Seiten 22-23, Absatz 6 Seiten 32-35, Absatz 8
• MATTHIAS FRAUENKRON, JOHANN-PETER MELDER, GÜNTHER RUIDER, ROLAND ROSSBACHER, HARTMUT HÖKE: "Ullmann's Encyclopedia of Industrial Chemistry, 6th ed, A2, Ethanolamines and Propanolamines" 2002, VCH , WEINHEIM , XP002525108 Absätze [2.2.], [2.4.], [4.2.], [4.4.]
• SRIVASAN SRIDHAR, RICHARD G CARTER: "Kirk-Othmer, Encyclopedia of Chemical Technology, 5th Ed, Vol. 8, Diamines and Higher Amines, Aliphatic" 2001, WILEY , NY , XP002525109 Absätze [4.1.], [4.2.], [009.]

## EP 2 197 830 B1

**Beschreibung**

Die vorliegende Erfindung betrifft ein Verfahren zur Herstellung von Aminen aus Zuckeralkoholen, wobei der Zuckeralkohol Sorbitol ist.

Die großtechnische Herstellung von technisch wichtigen Aminoalkanolen, wie Ethanolamin und Isopropanolamin, und deren Folgeprodukte, wie Ethylendiamin,1,2-Propylendiamin und Piperazin, geht in der Regel von Ethylenoxid oder Propylenoxid als $C_2$- bzw- $C_3$-Synthesebaustein aus.

So erfolgt die Synthese von Ethanolamin und Isopropanolamin durch Reaktion von Ammoniak mit Ethylenoxid bzw. Propylenoxid. Als weitere Produkte werden bei dieser Reaktion auch die entsprechenden Dialkanolamine und Trialkanolamine erhalten. Das Verhältnis von Monoalkanolaminen zu Di- und Trialkanolaminen kann durch die Einsatzmengen von Ammoniak zu Alkylenoxid gesteuert werden. Um einen höheren Anteil an Trialkanolaminen zu erhalten, können Mono- und Dialkanolamine in den Reaktor zurückgeführt werden.

In einer weiteren Reaktionsstufe können die so erhaltenen Monoalkanolamine durch Reaktion von Wasserstoff und Ammoniak zu Ethylendiamin bzw. 1,2-Propylendiamin weiter umgesetzt werden.

1,3-Diaminopropan ist großtechnisch durch Umsetzung von Ammoniak mit Acrylnitril und anschließender Hydrierung erhältlich, wobei Acrylnitril in der Regel großtechnisch durch Ammonoxidation des $C_3$-Bausteins Propen hergestellt wird.

Als alternative Rohstoffquelle zu den genannten petrochemischen Einsatzstoffen auf Ethen- bzw. Propenbasis, könnten Rohstoffe auf Basis von nachwachsenden Rohstoffen einen immer höheren Stellenwert erreichen. Eine wachsende Bedeutung könnten dabei in Zukunft Zuckeralkohole erlangen.

In US 2,016,962 wird die Umsetzung von reduzierenden Zuckern mit Wasserstoff und einem Aminierungsmittel (Ammoniak, primäre oder sekundäre Amine) in Gegenwart von Hydrogenierungskatalysatoren (reduzierten Nickel-Katalysatoren) beschrieben. Die Umsetzung sollte vorzugsweise zwischen 80 und 125°C durchgeführt werden, da Monosaccharide, wie Glucose, sich beim Erhitzen zersetzen. Aus Glucose bzw. Xylose wurden Glucamin bzw. Xylamin erhalten.

Kelkenberg et al. (H. Kelkenberg, Tenside Surfactants Detergents, 25(1988), Seiten 8-13) offenbart die Synthese von Glucamin durch Umsetzung von Glucose mit Amin bzw. Ammoniak zum N-Glucosid, welches anschließend zum Glucamin hydriert wird. Die Reaktion wird in Ni-Festbett durchgeführt, wobei die genauen Reaktionsbedingungen jedoch nicht spezifiziert sind. Zu scharfe Reaktionsbedingungen führen offenbarungsgemäß zu Spaltreaktionen und zur Bildung von Ethylendiamin und Ethanolamin.

EP-A2-0255033 beschreibt die Synthese von Isomaltinen aus Disacchariden durch katalytische reduktive Aminierung mit Hydrazin und Wasserstoff in Gegenwart von Nickel-Katalysatoren nach Raney. Die reduktive Aminierung wird bei 50°C und einem Druck von 100 bis 150 bar durchgeführt. EP-A2-0399488 offenbart ebenfalls die Herstellung von Polyhydroxyaminen aus Disacchariden durch reduktive Aminierung mit Hydrazin und Wasserstoff in Gegenwart von Nickel-Katalysatoren nach Raney.

In dem Übersichtsartikel von Fischer et al. (A. Fischer, T. Mallat, A. Baiker, Catalysis Today 37 (1997), Seiten 167-189) wird die Aminierung von Zuckern zusammenfassend dargestellt. Es wird darauf verwiesen, dass bei der Aminierung von Glucose das als Zwischenprodukt gebildete N-Glucosid insbesondere in Gegenwart von Wasser labil und zur Karamellisierungsreaktionen (Maillard-Reaktionen) neigt. Offenbarungsgemäß sind hohe Hydrogenierungsraten deshalb erforderlich, um die Lebensdauer des intermediär gebildeten Imins (N-Glucosid) zu verringern. Es wird weiterhin offenbart, dass andererseits schärfere Hydrierbedingungen zu Spaltprodukten, wie Ethanolamin und Diaminoethan, führen.

Der vorliegenden Erfindung lag die Aufgabe zu Grunde, Zuckeralkohole als Quelle für die Herstellung von Aminen zu nutzen. Es sollte ein Verfahren zur Verfügung gestellt werden, dass es sowohl erlaubt wichtige technische Amine als auch Spezialamine sowie Piperazinderivate zu erhalten, um den Rohstoff Zucker optimal verwerten zu können. Als technische Amine werden solche Amine bezeichnet, die üblicherweise auf Basis petrochemischer Rohstoffe erhalten werden, beispielsweise Monoamine, wie Methylamin, Ethylamin, i-Propylamin und n-Propylamin, Diamine, wie Ethylendiamin, 1,2-Propandiamin und 1,3-Propandiamin, Alkanolamine, wie Monoethanolamin, 2-Aminopropan-1-ol und 1-Aminopropan-2-ol, oder Piperazin.

Spezialamine sind Amine, die mindestens drei funktionelle Gruppen aufweisen, wobei mindestens eine funktionelle Gruppe eine Aminfunktion darstellt. Beispiele für solche Spezialamine sind 1,2-Diaminopropan-3-ol, Isomaltin und Glucosamin. Spezialamine leiten sich strukturell von Zuckeralkoholen bzw. deren Spaltprodukten ab.

Diese Verbindungen weisen eine hohe Anzahl von Funktionalitäten auf und können daher wichtige Zwischenprodukte in der Synthese von organischen Verbindungen, wie Pflanzenschutzmitteln, Pharmazeutika, Stabilisatoren etc. darstellen.

Derivate von Piperazin (Piperazinderivate), wie 2-Methylpiperazin oder 2,6-Dimethylpiperazin, können ebenfalls wichtige Synthesebausteine darstellen. Die Umsetzung von Zuckeralkoholen zu den genannten Verbindungen sollte nur wenige Reaktionsschritte beinhalten, um die Investitionskosten so gering wie möglich zu halten.

Durch einfach durchzuführende Einstellungen der Verfahrensbedingungen, wie beispielsweise Druck und Temperatur, Reaktionsdauer, Katalysatorbelastung, Variation des molaren Aminierungsmittel- zu Zuckeralkohol-Verhältnisses, als auch durch die Auswahl des eingesetzten Katalysators, sollte es zudem möglich sein die Zusammensetzung des Amingemisches innerhalb gewisser Grenzen zu regulieren, um somit besser auf Nachfrage- und Absatzschwankungen in bezug auf technische Amine, Spezialamine oder Piperazinderivate, reagieren zu können.

Zudem sollte die Bildung aromatischer Nebenprodukte, insbesondere von Pyrazinen und Pyrazinderivaten, gering gehalten werden, da diese die Ausbeute an technischen Aminen, Spezialaminen und Piperazinderivaten verringern. Die Bildung solcher heteroaromatischer Verbindungen aus Glucose ist zum Beispiel von Brandes et al. (P. Brandes, C. Stoehr, J. Prakt. Chem., 54 (1896), Seiten 481 bis 495) oder von Shu (C.-K. Shu, J. Agric. Food. Chem, 46 (1998), Seiten 1129 bis 1131) offenbart. Durch die Verwendung von Zuckeralkoholen anstelle der Zucker selbst wird überraschenderweise die Bildung von Heteroaromaten unterbunden.

Erfindungsgemäß wurde ein

Verfahren zur Herstellung von Aminen durch Umsetzung von Zuckeralkoholen mit Wasserstoff und einem Aminierungsmittel, ausgewählt aus der Gruppe Ammoniak, Methylamin, Dimethylamin, Ethylamin, Diethylamin, n-Propylamin, Di-npropyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentyl¬amin, iso-Pentylamin, n-Hexylamin, s-Hexylamin, iso-Hexylamin, Cyclohexylamin, Anilin, Toluidin, Piperidin, Morpholin und Pyrrolidin, in Gegenwart eines Katalysators bei einer Temperatur von 100°C bis 400°C und einem Druck von 1 bis 40 MPa

gefunden, wobei der Zuckeralkohol Sorbitol ist.

Die Umsetzung von Zuckeralkoholen in Gegenwart von Wasserstoff und eines Aminierungsmittels, ausgewählt aus der Gruppe Ammoniak, Methylamin, Dimethylamin, Ethylamin, Diethylamin, n-Propylamin, Di-npropyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin, wird im folgenden als hydrierende Aminierung von Zuckeralkoholen oder auch nur kurz als hydrierende Aminierung bezeichnet.

Als Edukte werden in die Reaktion Zuckeralkohole, Wasserstoff und ein Aminierungsmittel, ausgewählt aus der Gruppe bestehend aus Ammoniak, Methylamin, Dimethylamin, Ethylamin, Diethylamin, n-Propylamin, Di-npropyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentyl¬amin, iso-Pentylamin, n-Hexylamin, s-Hexylamin, iso-Hexylamin, Cyclohexylamin, Anilin, Toluidin, Piperidin, Morpholin und Pyrrolidin, eingesetzt,wobei der Zuckeralkohol Sorbitol ist

Die ins erfindungsgemäße Verfahren eingesetzten Zuckeralkohole werden bevorzugt durch Reduktion der Aldehyd- bzw. Carbonylgruppe von reduzierenden Zuckern erhalten.

So kann Glucitol (Sorbitol) durch Reduktion von Glucoseerhalten werden.

So erfolgt üblicherweise die Reduktion des voranstehend genannten Zuckers mit Wasserstoff bei hohen Drücken in Gegenwart von Hydrogenierungskatalysatoren, beispielsweise ruthenium-, nickel- oder cobalthaltigen Hydrogenierungskatalysatoren.

Verfahren zur Herstellung von, Sorbitol werden beispielsweise im Ullmann beschrieben (Ullmann's Encyclopedia of Industrial Chemistry, "Sugar Alcohols", Wiley-VCH-Verlag, Electronic Edition, 2007).

Bevorzugt wird Sorbitol gemäß der in EP-A-1412083 (BASF Aktiengesellschaft) oder EP-A-1399255 (BASF Aktiengesellschaft) beschriebenen Verfahrens durch Reduktion von Glucose mit Wasserstoff an Ru-Katalysatoren hergestellt.

Als weiterer Einsatzstoff wird Wasserstoff in das Verfahren eingesetzt.

Das Aminierungsmittel wird ausgewählt aus der Gruppe bestehend aus Ammoniak,

Methylamin, Dimethylamin, Ethylamin, Diethylamin, n-Propylamin, Di-npropyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Vorzugsweise werden jedoch großtechnisch verfügbare und kostengünstige Aminierungsmittel, wie Ammoniak, Methylamin oder Diethylamin eingesetzt.

Besonders bevorzugt wird Ammoniak als Aminierungsmittel eingesetzt.

Die im erfindungsgemäßen Verfahren eingesetzten Katalysatoren enthalten ein oder mehrere Metalle der Gruppen 8 und/oder 9 und/oder 10 und/oder 11 des Periodensystems der Elemente (Periodensystem in der IUPAC-Fassung vom 22.06.2007, http://www.iupac.org/reports/periodic_table/IUPAC_Periodic_Table-22Jun07b.pdf). Beispiele für solche Metalle sind Cu, Co, Ni und/oder Fe, sowie auch Edelmetalle wie Rh, Ir, Ru, Pt, Pd, sowie Re.

Die oben genannten Metalle können in Form von Metallnetzen oder Gittern eingesetzt werden.

In einer bevorzugten Ausführungsform werden die Metalle in Form von Schwamm- oder Skelettkatalysatoren nach Raney in das erfindungsgemäße Verfahren eingesetzt. Besonders bevorzugt werden Nickel- und/oder Cobalt-Katalysatoren nach Raney eingesetzt.

Die Herstellung von Nickel- bzw. Cobalt-Katalysatoren nach Raney erfolgt üblicherweise durch Behandlung einer Aluminium-Nickel- bzw. einer Aluminium-Cobalt-Legierung mit konzentrierter Natronlauge, wobei das Aluminium ausgelaugt wird und ein metallischer Nickel- bzw. Cobaltschwamm entsteht. Die Herstellung von Katalysatoren nach Raney ist beispielsweise im Handbook of Heterogeneous Catalysis beschrieben (M. S. Wainright in G. Ertl, H. Knözinger, J. Weitkamp (eds.), Handbook of Heterogeneous Catalysis, Vol. 1, Wiley-VCH, Weinheim, Germany 1997, Seite 64 ff.). Solche Katalysatoren sind beispielsweise als Raney®-Katalysatoren von der Fa. Grace oder als Sponge Metal®-Katalysatoren der Johnson Matthey erhältlich.

Die in das erfindungsgemäße Verfahren einsetzbaren Katalysatoren können auch durch Reduktion von sogenannten Katalysatorvorläufern hergestellt werden.

Der Katalysatorvorläufer enthält eine aktive Masse, die eine oder mehrere katalytisch aktive Komponenten und optional ein Trägermaterial enthält.

Bei den katalytisch aktiven Komponenten handelt es sich um sauerstoffhaltige Verbindungen der Metalle der Gruppen 8 und/oder 9 und/oder 10 und/oder 11 des Periodensystems der Elemente (Periodensystem in der IUPAC-Fassung vom 22.06.2007), beispielsweise um deren Metalloxide bzw. Hydroxide. (ggf. Beispiele), wie $CoO$, $NiO$, $Mn_3O_4$, $CuO$, $RuO(OH)_x$ und/oder deren Mischoxide, wie $LiCoO_2$.

Die Masse der aktiven Masse ist die Summe aus der Masse des Trägermaterials und aus der Masse der katalytisch aktiven Komponenten.

Die in dem Verfahren eingesetzten Katalysatorvorläufer können neben der aktiven Masse Verformungsmittel, wie Graphit, Stearinsäure, Phosphorsäure oder weitere Verarbeitungshilfsmittel enthalten.

Die in dem Verfahren eingesetzten Katalysatorvorläufer können weiterhin ein oder mehrere Dotierelemente (Oxidationsstufe 0) oder deren anorganische oder organische Verbindungen, ausgewählt aus den Gruppen 1 bis 14 des Periodensystems, enthalten. Beispiele für solche Elemente bzw. deren Verbindungen sind: Übergangsmetalle, wie Mn bzw. Manganoxide, Re bzw. Rheniumoxide, Cr bzw. Chromoxide, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat, Zink bzw. Zinkoxide, Silber bzw. Silberoxide, Lanthanide, wie Ce bzw. $CeO_2$ oder Pr bzw. $Pr_2O_3$, Alkalimetalloxide, wie $K_2O$, Alkalimetallcarbonate, wie $Na_2CO_3$ und $K_2CO_3$, Erdalkalimetalloxide, wie SrO, Erdalkalimetallcarbonate, wie $MgCO_3$, $CaCO_3$, $BaCO_3$, Phosphorsäureanhydride und Boroxid ($B_2O_3$).

Im erfindungsgemäßen Verfahren werden die Katalysatorvorläufer bevorzugt in Form von Katalysatorvorläufern eingesetzt, die nur aus katalytisch aktiver Masse, gegebenenfalls einem Verformungshilfsmittel (wie z. B. Graphit oder Stearinsaure), falls der Katalysator als Formkörper eingesetzt wird und gegebenenfalls ein oder mehreren Dotierelementen bestehen, aber darüber hinaus keine weiteren katalytisch aktiven Begleitstoffe enthalten. In diesem Zusammenhang wird das Trägermaterial als zur katalytisch aktiven Masse gehörig gewertet.

Die im folgenden angegebenen Zusammensetzungen beziehen sich auf die Zusammensetzung des Katalysatorvorläufers nach dessen letzter Wärmebehandlung, welche im Allgemeinen eine Calcinierung darstellt, und vor dessen Reduktion mit Wasserstoff.

Der Anteil der aktiven Masse bezogen auf die Gesamtmasse des Katalysatorvorläufers beträgt üblicherweise 70 Gew.-% oder mehr, bevorzugt 80 bis 100 Gew.-%, besonders bevorzugt 90 bis 99 Gew.-%, insbesondere 92 bis 98 Gew.-%.

In einer bevorzugten Ausführungsform enthält die aktive Masse des Katalysatorvorläufers kein Trägermaterial.

Die aktive Masse von Katalysatorvorläufern, die kein Trägermaterial enthalten, enthält bevorzugt eine oder mehrere aktive Komponenten ausgewählt aus der Gruppe bestehend aus $CoO$, $NiO$, $Mn_3O_4$, $CuO$, $RuO(OH)_x$ und $LiCoO_2$.

Besonders bevorzugterweise enthält die aktive Masse von Katalysatorvorläufern, die kein Trägermaterial enthalten, NiO und/oder CoO.

Solche Katalysatorvorläufer sind beispielsweise

in der Patenanmeldung mit dem Anmeldezeichen PCT/EP2007/052013 offenbarte Katalysatoren, die vor der Reduktion mit Wasserstoff a) Kobalt und b) ein oder mehrere Elemente der Alkalimetallgruppe, der Erdalkalimetallgruppe, der Gruppe der Seltenen Erden oder Zink oder Mischungen daraus enthalten, wobei die Elemente a) und b) zumindest zum Teil in Form ihrer Mischoxide vorliegen, beispielsweise $LiCoO_2$, oder

in EP-A-0636409 offenbarte Katalysatoren, deren katalytisch aktive Masse vor der Reduktion mit Wasserstoff 55 bis 98 Gew.-% Co, berechnet als CoO, 0,2 bis 15 Gew.-% Phosphor, berechnet als $H_3PO_4$, 0,2 bis 15 Gew.-% Mangan, berechnet als $MnO_2$, und 0,2 bis 15 Gew.-% Alkali, berechnet als $M_2O$ (M=Alkali), enthält, oder

in EP-A-0742045 offenbarte Katalysatoren, deren katalytisch aktive Masse vor der Reduktion mit Wasserstoff 55 bis 98 Gew.-% Co, berechnet als CoO, 0,2 bis 15 Gew.-% Phosphor, berechnet als $H_3PO_4$, 0,2 bis 15 Gew.-% Mangan, berechnet als $MnO_2$, und 0,05 bis 5 Gew.-% Alkali, berechnet als $M_2O$ (M=Alkali), enthält.

In einer weiteren bevorzugten Ausführungsform enthält die aktive Masse - zusätzlich zu den katalytisch aktiven Komponenten - Trägermaterial.

Katalysatorvorläufer, die Trägermaterial enthalten, können ein oder mehrere katalytisch aktive Komponenten enthalten, bevorzugt CoO, NiO, $Mn_3O_4$, CuO und/oder sauerstoffhaltige Verbindungen von Rh, Ru und/oder Ir. Besonders bevorzugt enthält die aktive Masse von Katalysatorvorläufern, die Trägermaterial enthalten, NiO und/oder CoO.

Als Trägermaterial werden bevorzugt Kohlenstoff, wie Graphit, Russ und/oder Aktivkohle, Aluminiumoxid (gamma, delta, theta, alpha, kappa, chi oder Mischungen daraus), Siliziumdioxid, Zirkoniumdioxid, Zeolithe, Alumosilicate, etc. sowie Mischungen aus diesen Trägermaterialien verwendet.

Der Anteil an Trägermaterial an der aktiven Masse kann über einen breiten Bereich je nach gewählter Herstellungsmethode variieren.

Bei Katalysatorvorläufern, die durch Tränkung (Imprägnierung) hergestellt werden, beträgt der Anteil an Trägermaterial an der aktiven Masse in der Regel mehr als 50 Gew.-%, bevorzugt mehr als 75 Gew.-% uns besonders bevorzugt mehr als 85 Gew.-%.

Bei Katalysatorvorläufern, die durch Fällungsreaktionen, wie Mischfällung oder Auffällung, hergestellt werden, beträgt der Anteil an Trägermaterial an der aktiven Masse in der Regel im Bereich von 10 bis 90 Gew.-%, bevorzugt im Bereich von 15 bis 80 Gew.-% und besonders bevorzugt im Bereich von 20 bis 70 Gew.-%.

Solche Katalysatorvorläufer, die durch Fällungsreaktionen erhalten werden, sind beispielsweise

in EP-A-696572 offenbarte Katalysatoren, deren katalytisch aktive Masse vor der Reduktion mit Wasserstoff 20 bis 85 Gew.-% $ZrO_2$, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als $MoO_3$, und 0 bis 10 Gew.-% sauerstoffhaltige Verbindungen des Aluminiums und/oder Mangans, berechnet als $Al_2O_3$ bzw. $MnO_2$, enthält, beispielsweise der in loc. cit, Seite 8, offenbarte Katalysator mit der Zusammensetzung 31,5 Gew.-% $ZrO_2$, 50 Gew.-% NiO, 17 Gew.-% CuO und 1,5 Gew.-% $MoO_3$,

in EP-A-963 975 offenbarte Katalysatoren, deren katalytisch aktive Masse vor der Reduktion mit Wasserstoff 22 bis 40 Gew.-% $ZrO_2$, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhaltnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als $Al_2O_3$ bzw. $MnO_2$, und keine sauerstoffhaltigen Verbindungen des Molybdans enthält, beispielsweise der in loc. cit., Seite 17, offenbarte Katalysator A mit der Zusammensetzung 33 Gew.-% Zr, berechnet als $ZrO_2$, 28 Gew.-% Ni, berechnet als NiO, 11 Gew.-% Cu, berechnet als CuO und 28 Gew.-% Co, berechnet als CoO,

in DE-A-2445303 offenbarte kupferhaltigen Katalysatoren, z.B. der im dortigen Beispiel 1 offenbarte kupferhaltige Fällungskatalysator, der durch Behandlung einer Lösung von Kupfernitrat und Aluminiumnitrat mit Natriumbicarbonat und anschließendem Waschen, Trocknen und Tempern des Präzipitats hergestellt wird und eine Zusammensetzung von ca. 53 Gew.-% CuO und ca. 47 Gew.-% $Al_2O_3$ aufweist, oder

in WO 96/36589 offenbarte Katalysatoren, insbesondere solche, die Ir, Ru und/oder Rh und als Trägermaterial Aktivkohle enthalten.

Die Katalysatorvorläufer können nach bekannten Verfahren, z.B. durch Fällung, Auffällung, Imprägnierung, hergestellt werden.

In einer bevorzugten Ausführungsform werden Katalysatorvorläufer in das erfindungsgemäße Verfahren eingesetzt, die durch Tränkung (Imprägnierung) von Trägermaterialien hergestellt werden (getränkte Katalysatorvorläufer).

Die Trägermaterialien, die bei der Imprägnierung eingesetzt werden, können beispielsweise in Form von Pulvern oder Formkörpern, wie Strängen, Tabletten, Kugeln oder Ringen, eingesetzt werden. Für Wirbelbettreaktoren geeignetes Trägermaterial wird vorzugsweise durch Sprühtrocknung erhalten.

Als Trägermaterialien kommen beispielsweise Kohlenstoff, wie Graphit, Russ und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Die Tränkung der obengenannten Trägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der oben genannten Elemente in Betracht. Im Anschluss wird das getränkte Trägermaterial in der Regel getrocknet und ggf. calciniert.

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Metallsalzen beaufschlagt werden soll.

Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

In einer weiteren bevorzugten Ausführungsform werden Katalysatorvorläufer über eine gemeinsame Fällung (Mischfällung) aller ihrer Komponenten hergestellt. Dazu wird in der Regel ein lösliches Metallsalz der entsprechenden Metalloxide und ggf. eine lösliche Verbindung eines Trägermaterials in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Metallsalze der entsprechenden Metalloxide kommen üblicherweise die entsprechenden Nitrate, Sulfate, Acetate oder Chloride der Metalle der Gruppen 8 und/oder 9 und/oder 10 und/oder 11 des Periodensystems der Elemente (Periodensystem in der IUPAC-Fassung vom 22.06.2007) in Betracht. Beispiele für solche Metalle sind Cu, Co, Ni und/oder Fe, sowie auch Edelmetalle wie Rh, Ir, Ru, Pt, Pd, sowie Re. Als wasserlösliche Verbindungen eines Trägermaterials werden in der Regel wasserlösliche Verbindungen von Al, Zr, Si etc., beispielsweise die wasserlöslichen Nitrate, Sulfate, Acetate oder Chloride dieser Elemente, verwendet.

Katalysatorvorläufer können weiterhin durch Auffällung hergestellt werden.

Unter Auffällung wird eine Herstellmethode verstanden, bei der ein schwer lösliches oder unlösliches Trägermaterial in einer Flüssigkeit suspendiert wird und nachfolgend lösliche Metallsalze der entsprechenden Metalloxide zugegeben werden, welche dann durch Zugabe eines Fällungsmittels auf den suspendierten Träger aufgefällt werden (z.B. beschrieben in EP-A2-1 106 600, Seite 4, und A. B. Stiles, Catalyst Manufacture, Marcel Dekker, Inc., 1983, Seite 15).

Als schwer- bzw. unlösliche Trägermaterialien kommen beispielsweise Kohlenstoffverbindungen, wie Graphit, Russ und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Das Trägermaterial liegt in der Regel als Pulver oder Splitt vor.

Als Flüssigkeit, in der das Trägermaterial suspendiert wird, wird üblicherweise Wasser eingesetzt.

Als lösliche Metallsalze der entsprechenden Metalloxide kommen in der Regel die entsprechenden Nitrate, Sulfate, Acetate oder Chloride der Metalle der Gruppen 8 und/oder 9 und/oder 10 und/oder 11 des Periodensystems der Elemente (Periodensystem in der IUPAC-Fassung vom 22.06.2007). Beispiele für solche Metalle sind Cu, Co, Ni und/oder Fe, sowie auch Edelmetalle wie Rh, Ir, Ru, Pt, Pd und Re.

Bei den Fällungsreaktionen ist im Allgemeinen die Art der verwendeten löslichen Metallsalze nicht kritisch. Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zur Störungen führen, sei es, indem sie unerwünschte Fällungsreaktionen verursachen oder indem sie durch Komplexbildung die Fällung erschweren oder verhindern.

Üblicherweise werden bei den Fällungsreaktionen die löslichen Verbindungen durch Zugabe eines Fällungsmittels als schwer- oder unlösliche, basische Salzen gefällt.

Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.

Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100°C, besonders 30 bis 90°C, insbesondere bei 50 bis 70°C, durchgeführt werden.

Die bei den Fällungsreaktionen erhaltenen Niederschläge sind im allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im allgemeinen bei 80 bis 200°C, vorzugsweise 100 bis 150°C, getrocknet und anschließend calciniert.

Die Calcinierung wird im allgemeinen bei Temperaturen zwischen 300 und 800°C, vorzugsweise 400 bis 600°C, insbesondere bei 450 bis 550°C ausgeführt.

Nach der Calcinierung werden die durch Fällungsreaktionen erhaltenen Katalysatorvorläufer üblicherweise konditioniert.

Die Konditionierung kann beispielsweise dadurch erfolgen, dass man den Fällungskatalysator durch Vermahlen auf eine bestimmte Korngröße einstellt.

Nach der Vermahlung kann der durch Fällungsreaktionen erhaltenen Katalysatorvorläufer mit Formhilfsmitteln wie Graphit, oder Stearinsäure vermischt werden und zu Formkörpern weiterverarbeitet werden.

Gängige Verfahren der Formgebung sind beispielsweise im Ullmann [Ullmann's Encyclopedia Electronic Release 2000, Kapitel: "Catalysis and Catalysts", Seiten 28-32] und von Ertl et al. [Ertl, Knözinger, Weitkamp, Handbook of Heterogenoeous Catalysis, VCH Weinheim, 1997, Seiten 98 ff] beschrieben.

Wie in den genannten Literaturstellen beschrieben, können durch den Prozess der Formgebung Formkörper in jeglicher Raumform, z.B. rund, eckig, länglich oder dergleichen, z.B. in Form von Strängen, Tabletten, Granulat, Kugeln, Zylindern oder Körnern erhalten werden. Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Nach der Konditionierung bzw. Formgebung erfolgt in der Regel eine Temperung. Die Temperaturen bei der Temperung entsprechen üblicherweise den Temperaturen bei der Calcinierung.

Die durch Fällungsreaktionen erhaltenen Katalysatorvorläufer enthalten die katalytisch aktiven Komponenten in Form eines Gemisches ihrer sauerstoffhaltigen Verbindungen, d.h. insbesondere als Oxide, Mischoxide und/oder Hydroxide. Die so hergestellten Katalysatorvorläufer können als solche gelagert werden.

Vor ihrem Einsatz als Katalysatoren zur hydrierenden Aminierung von Zuckeralkoholen werden Katalysatorvorläufer, die wie voranstehend beschrieben durch Imprägnierung oder Fällung erhalten wurden, werden in der Regel nach der Calcinierung bzw. Konditionierung durch Behandlung mit Wasserstoff vorreduziert.

Zur Vorreduktion werden die Katalysatorvorläufer im allgemeinen zunächst bei 150 bis 200°C über einen Zeitraum von 12 bis 20 Stunden einer Stickstoff-Wasserstoff-Atmosphäre ausgesetzt und anschließend noch bis zu ca. 24 Stunden bei 200 bis 400°C in einer Wasserstoffatmosphäre behandelt. Bei dieser Vorreduktion wird ein Teil der in den Katalysatorvorläufern vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

In einer bevorzugten Ausführungsform wird die Vorreduktion des Katalysatorvorläufers in demselben Reaktor vorgenommen, in dem anschließend die hydrierende Aminierung der Zuckeralkohole durchgeführt wird.

Der so gebildete Katalysator kann nach der Vorreduktion unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Katalysator eingesetzt wird. Der Katalysator kann nach der Vorreduktion aber auch mit einem Sauerstoff enthaltenden Gasstrom wie Luft oder einem Gemisch von Luft mit Stickstoff passiviert, d. h. mit einer schützenden Oxidschicht versehen werden.

Die Lagerung der Katalysatoren, die durch Vorreduktion von Katalysatorvorläufern erhalten wurden, unter inerten Substanzen oder die Passivierung des Katalysators ermöglichen eine unkomplizierte und ungefährliche Handhabung und Lagerung des Katalysators. Gegebenenfalls muss der Katalysator vor Beginn der eigentlichen Reaktion dann von der inerten Flüssigkeit befreit werden bzw. die Passivierungsschicht z. B. durch Behandlung mit Wasserstoff oder einem Wasserstoff enthaltenden Gas aufgehoben werden.

Der Katalysator kann vor Beginn der Hydroaminierung von der inerten Flüssigkeit bzw. Passivierungsschicht befreit werden. Dies geschieht beispielsweise durch die Behandlung mit Wasserstoff oder einem Wasserstoff enthaltendem Gas. Bevorzugt wird die Hydroaminierung direkt nach der Reduktion des Katalysatorvorläufers im selben Reaktor vorgenommen, in dem auch die Reduktion erfolgte.

Katalysatorvorläufer können jedoch auch ohne Vorreduktion in das Verfahren eingesetzt werden, wobei sie dann unter den Bedingungen der hydrierenden Aminierung durch dem im Reaktor vorhandenen Wasserstoff reduziert werden, wobei sich der Katalysator in der Regel in situ bildet.

In dem erfindungsgemäßen Verfahren wird Zuckeralkohol üblicherweise in flüssiger Phase (Flüssigphase), d.h. geschmolzen oder in einem Lösungsmittel oder Verdünnungsmittel gelöst bzw. suspendiert reduktiv aminiert.

Als Lösungsmittel oder Verdünnungsmittel kommen insbesondere solche in Betracht, die das Edukt möglichst vollständig zu lösen vermögen oder sich mit diesen vollständig mischen und die unter den Verfahrensbedingen inert sind.

Beispiele für geeignete Lösungs- und Verdünnungsmittel sind Wasser und aliphatische Alkohole, insbesondere $C_{1-8}$-Alkohole, besonders $C_{1-4}$-Alkohole, wie Methanol, Ethanol, n- oder Isopropanol, n-, 2-, iso- oder tert.-Butanol.

Besonders bevorzugt wird die reduktive Aminierung von Zuckeralkoholen in Gegenwart von Wasser als Lösungsmittel oder Verdünnungsmittel durchführt.

Die Konzentration an Zuckeralkohol in der flüssigen, lösungsmittel- oder verdünnungsmittel-haltigen Phase liegt bevorzugt im Bereich von 10 bis 80 Gew.-%, besonders bevorzugt 30 bis 70 Gew.-%, jeweils bezogen auf das Gesamtgewicht der Lösung bzw. Suspension (ohne Katalysator).

Das Verfahren kann kontinuierlich, diskontinuierlich oder semi-kontinuierlich durchgeführt werden. Bevorzugt ist eine kontinuierliche Fahrweise, wobei der Katalysator bevorzugt als Festbett im Reaktor angeordnet ist. Es ist auch möglich die hydrierende Aminierung in einem Rührautoklaven, einer Blasensäule, einem Umlaufreaktor wie etwa einer Strahlschlaufe oder einem Festbettreaktor durchzuführen.

Bei der diskontinuierlichen reduktiven Aminierung wird Zuckeralkohol üblicherweise als Schmelze, Lösung oder Suspension, bevorzugt als Lösung, in einem geeigneten Lösungs- oder Verdünnungsmittel (siehe oben) im Reaktor vorgelegt; das Katalysatormaterial wird in der Regel in der flüssigen Phase suspendiert. Um einen hohen Umsatz und hohe Selektivität zu gewährleisten, muss die Schmelze, Lösung oder Suspension des Zuckeralkohols sowie der Katalysator und Wasserstoffgas in der Regel gut gemischt werden, z.B. durch einen Turbinenrührer in einem Autoklaven. Das suspendierte Katalysatormaterial kann mit Hilfe gebräuchlicher Techniken eingebracht und wieder abgetrennt werden (Sedimentation, Zentrifugation, Kuchenfiltration, Querstromfiltration). Der Katalysator kann einmal oder mehrmals verwendet werden. Die Katalysatorkonzentration beträgt vorteilhaft 0,1 bis 20 Gew.-%, bevorzugt 0,5 bis 10 Gew.-%, insbesondere 1 bis 5 Gew.-%, jeweils bezogen auf das Gesamtgewicht der Lösung bzw. Suspension (Gesamtgewicht mit Katalysator). Die mittlere Katalysatorpartikelgröße beträgt vorteilhaft im Bereich von 0,001 bis 1 mm, bevorzugt im Bereich von 0,005 bis 0,5 mm, insbesondere 0,01 bis 0,25 mm.

Bei der kontinuierlichen reduktiven Aminierung leitet man Zuckeralkohole üblicherweise als Schmelze bzw. als Lösung oder Suspension, bevorzugt als Lösung in einem geeigneten Lösungs- oder Verdünnungsmittel (siehe oben), in flüssiger Phase inklusive Wasserstoff und Aminierungsmittel (Ammoniak oder Amin) über den Katalysator, der sich bevorzugt in einem (bevorzugt von außen) beheizten Festbettreaktor befindet. Es ist dabei sowohl eine Rieselfahrweise als auch eine Sumpffahrweise möglich. Die Katalysatorbelastung liegt im allgemeinen im Bereich von 0,05 bis 5, bevorzugt 0,1 bis 2, besonders bevorzugt 0,2 bis 0,6 kg Zuckeralkohol pro Liter Katalysator (Schüttvolumen) und Stunde.

Es kann auch eine kontinuierliche Suspensionsfahrweise angewendet werden, wie z.B. beschrieben in EP-A2-1 318 128 (BASF AG) oder in FR-A-2 603 276 (Inst. Français du Pétrole).

Bei der Durchführung des Verfahrens in der Flüssigphase beträgt der Druck im Allgemeinen von 1 bis 40 MPa (10-400 bar), bevorzugt 1,5 bis 35 MPa, besonders bevorzugt 5 bis 30 MPa.

Die Temperatur beträgt im Allgemeinen von 100 bis 400°C, bevorzugt 150 bis 300°C, besonders bevorzugt 180 bis 250°C. Es ist zweckmäßig, die Reaktanden bereits vor der Zuführung in das Reaktionsgefäß zu erwärmen, und zwar bevorzugt auf die Reaktionstemperatur.

Das Aminierungsmittel wird bevorzugt in der 0,90- bis 250-fachen molaren Menge, bevorzugt in der 1,0- bis 100-fachen molaren Menge insbesondere in der 1,0- bis 10-fachen molaren Menge, jeweils bezogen auf den Zuckeralkohol eingesetzt.

Speziell Ammoniak wird im allgemeinen mit einem 1,5 bis 250-fachen, bevorzugt 2 bis 100-fachen, insbesondere 2 bis 10-fachen molaren Überschuss pro Mol Zuckeralkohol eingesetzt.

Höhere Überschusse sowohl an Ammoniak als auch an primären oder sekundären Aminen sind möglich.

Der Wasserstoff wird der Reaktion im allgemeinen in einer Menge von 5 bis 400 l, bevorzugt in einer Menge von 150 bis 600 l pro Mol Zuckeralkohol zugeführt, wobei die Literangaben jeweils auf Normalbedingungen umgerechnet wurden (S.T.P.).

Bei der Durchführung des Verfahrens in der Flüssigphase ist die Anwendung höherer Temperaturen und höherer Gesamtdrücke möglich. Der Druck im Reaktionsgefäß, welcher sich aus der Summe der Partialdrücke des Aminierungsmittels, des Zuckeralkohols und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl bei der kontinuierlichen Durchführung des Verfahrens in der Flüssigphase als auch bei der kontinuierlichen Durchführung des Verfahrens in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesondere 40 bis 50 Volumenteile betragen.

Das im Zuge der Umsetzung gebildete Reaktionswasser (jeweils ein Mol pro Mol umgesetzte Alkoholgruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ oder extraktiv.

Üblicherweise wird das Aminierungsmittel zusammen mit Zuckeralkoholen und Wasserstoff zu Beginn der Reaktion zugegeben. In einer bevorzugten Ausführungsform ist es auch möglich das Aminierungsmittel dem Reaktionsgemisch erst dann zuzuführen, nach dem ein Teil der Zuckeralkohole mit Wasserstoff in Gegenwart einer der voranstehend aufgeführten Katalysatoren umgesetzt wurde. In dieser bevorzugten Ausführungsform des Verfahrens wird in der Regel in zwei Stufen durchgeführt. In der ersten Stufe werden üblicherweise erst die Zuckeralkohole in Gegenwart von Wasserstoff und einem oder mehreren der voranstehend aufgeführten Katalysatoren unter den voranstehend genannten Reaktionsbedingungen umgesetzt. In der zweiten Reaktionsstufe wird üblicherweise zu dem Reaktionsgemisch aus der ersten Stufe Aminierungsmittel in den voranstehend aufgeführten Mengen (Molverhältnissen) zugegeben. In der zweiten Reaktionsstufe kann derselbe Katalysator eingesetzt werden, der auch in der ersten Reaktionsstufe eingesetzt wurde, es kann aber auch ein anderer der voranstehend aufgeführten Katalysatoren eingesetzt werden. Es ist auch möglich, jeweils die erste und/oder die zweite Stufe in weitere Teilstufen zu unterteilen, wobei in jeder Teilstufe derselbe oder ein anderer der voranstehend aufgeführten Katalysatoren eingesetzt werden kann. Es ist möglich das Aminierungsmittel zu Beginn der zweiten Stufe zuzuführen oder die Zuführung des Aminierungsmittel auf mehrere Teilstufen der zweiten Stufe aufzuteilen.

Die mehrstufige Umsetzung von Zuckeralkoholen mit Wasserstoff und Aminierungsmittel in Gegenwart von Katalysatoren kann diskontinuierlich, semikontinuierlich oder kontinuierlich erfolgen.

Bei der kontinuierlichen Durchführung werden die Reaktionsstufen bzw. Teilstufen bevorzugt in getrennten Reaktionsräumen, bevorzugt in Festbettreaktoren, durchgeführt. In der ersten Reaktionsstufe erfolgt üblicherweise zunächst die Umsetzung der Zuckeralkohole mit Wasserstoff in Gegenwart einer der voranstehend aufgeführten Katalysatoren. Die erste Stufe wird bevorzugt in einem Festbettreaktor durchgeführt, wobei das Aminierungsmittel bevorzugt am Eingang des zweiten Festbettreaktors zudosiert wird.

Bei der diskontinuierlichen Reaktionsführung können die Reaktionsstufen in einem oder mehreren Reaktoren durchgeführt werden.

So kann beispielsweise zunächst die Umsetzung von Zuckeralkohol mit Wasserstoff in Gegenwart eines Katalysators in einem Reaktor erfolgen und nach dem ein Teil des Zuckeralkohols umgesetzt wurde, anschließend die Zugabe des Aminierungsmittels in denselben Reaktor erfolgen. Üblicherweise wird bei dieser Ausführung, derselbe Katalysator in der zweiten Stufe eingesetzt, der auch in der ersten Stufe verwendet wurde. Die Zugabe des Aminierungsmittels in die zweite Stufe kann semikontinuierlich erfolgen. Es ist auch möglich die diskontinuierliche zweistufige Durchführung in zwei Reaktoren, beispielsweise zwei Rührkesseln oder Rührautoklaven, durchzuführen. Im ersten Reaktor wird in der Regel zunächst die Umsetzung von Wasserstoff und Zuckeralkohol in Gegenwart eines Katalysators durchgeführt. Nach dem ein Teil des Zuckeralkohols umgesetzt wurde, wird üblicherweise das Reaktionsgemisch in den zweiten Reaktor geleitet, in dem die Zugabe des Aminierungsmittels erfolgt. Der Katalysator, der im ersten Reaktor verwendet wurde wird üblicherweise von dem Reaktionsgemisch vor der Einleitung in den zweiten Reaktor abgetrennt, beispielsweise durch Filtration. In dieser Variante wird üblicherweise im ersten Reaktor ein anderer Katalysator verwendet als im zweiten Reaktor.

Mit dem erfindungsgemäßen Verfahren können Amine aus Zuckeralkoholen, Wasserstoff und einem Amin, ausgewählt aus der Gruppe Ammoniak, Methylamin, Dimethylamin, Ethylamin, Diethylamin, n-Propylamin, Di-npropyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentyl¬amin, iso-Pentylamin, n-Hexylamin, s-Hexylamin, iso-Hexylamin, Cyclohexylamin, Anilin, Toluidin, Piperidin, Morpholin und Pyrrolidin, hergestellt werden.

In der Regel wird ein Amingemisch, enthaltend

ein oder mehrere Monoamine ausgewählt aus der Gruppe bestehend aus Methyl-amin, Ethylamin, i-Propylamin und n-Propylamin, und/oder

ein oder mehrere Diamine ausgewählt aus der Gruppe bestehend aus Ethylendiamin, 1,2-Propandiamin und 1,3-Propandiamin, 1,2-Butandiamin, 1,3-Butandiamin, 1,4-Butandiamin und/oder

ein oder mehrere Alkanolamine, ausgewählt aus der Gruppe bestehend aus Monoethanolamin, 2-Aminopropan-1-ol und 1-Aminopropan-2-ol, und/oder ein oder mehrerer zuckeralkoholanaloge Spezialamine, ausgewählt aus der Gruppe bestehend aus 1,2,3-Triaminopropan, 1,3-Diaminopropan-2-ol, 1,2-Diaminopropan-3-ol, 1-Aminopropandiol, 2-Aminopropandiol, Glucosamin und Isomaltin und/oder Piperazin, und/oder

ein oder mehrere Piperazinderivate, ausgewählt aus der Gruppe bestehend aus aus aus 2-Methylpiperazin, 2,6-Dimethylpiperazin, 2,5-Dimethylpiperazin, 2,5-Bis(Aminomethyl)-piperazin, 2,6-Bis(Aminomethyl)-piperazin, 2-Aminomethyl-5-methyl-piperazin und 2-Aminomethyl-6-methyl-piperazin,

erhalten.

Spezialamine sind Amine, die mindestens 3 funktionelle Gruppen aufweisen, wobei mindestens eine funktionelle Gruppe eine Aminfunktion darstellt, beispielsweise eine primäre Aminogruppe, eine sekundäre Aminogruppe oder eine tertiäre Aminogruppe. Spezialamine leiten sich strukturell von Zuckeralkoholen bzw. deren Spaltprodukten ab, beispielsweise 1,2-Diaminopropan-3-ol, Glucosamin, Isomaltin oder andere Zuckeralkohole, bei denen eine oder mehrere Hydroxygruppen gegen eine Aminogruppe substituiert wurde.

Die Zusammensetzung des Reaktionsaustrags kann durch den Zuckeralkohol-Umsatz, die Reaktionstemperatur sowie die Zusammensetzung des Katalysators beeinflusst werden

Beispielsweise kann die Zusammensetzung des eingesetzten Katalysators die Zusammensetzung der Amine im Reaktionsaustrag beeinflussen.

In einer besonderen Ausführungsform des erfindungsgemäßen Verfahren wird als Katalysator ein Katalysator eingesetzt, der Ni und/oder Co enthält, beispielsweise ein Nickel- bzw. Cobalt-Katalysator nach Raney oder ein Katalysator, der durch Reduktion eines Katalysatorvorläufers erhalten wurde und dessen aktive Masse vor der Reduktion mit Wasserstoff als katalytische aktive Komponente NiO und/oder CoO enthält. Solche Katalysatoren weisen im Allgemeinen eine hohe Aktivität auf und begünstigen insbesondere die Bildung von Alkanolaminen, Diaminen, Spezialaminen und/oder Piperazinderivaten.

Bei Katalysatoren, die durch Reduktion von Katalysatorvorläufern hergestellt werden, begünstigt insbesondere das Vorhandensein der katalytisch aktiven Komponente NiO und/oder CoO die Bildung von Spezialaminen und/oder Piperazin bzw. Piperazinderivaten.

In einer weiteren bevorzugten Ausführungsform werden Schwammkatalysatoren nach Raney mit Ni oder Co als Aktivmetall in das erfindungsgemäße Verfahren eingesetzt. Schwammkatalysatoren nach Raney mit Ni oder Co als Aktivmetall begünstigen in der Regel die Bildung von acyclischen Diaminen bzw. Spezialaminen. Da diese Katalysatoren eine besonders hohe Aktivität aufweisen, wird in der Regel bereits bei niedrigen Temperaturen oder kurzen Reaktionszeiten ein hoher Zuckeralkohol-Umsatz erreicht eine hohe Ausbeute an Spezialminen bzw. technisch bedeutsamen Aminen wie Propandiamin und Ethylendiamin bei der Verwendung von Ammoniak als Aminierungsmittel erhalten.

In einer ebenfalls bevorzugten Ausführungsform werden Katalysatoren, die ein Metall oder mehrere Metalle der 5. Periode der Gruppen 8 und/oder 9 und/oder 10 und/oder 11 des Periodensystems der Elemente enthalten, vorzugsweise Ru und/oder Rh, in das erfindungsgemäße Verfahren eingesetzt. Die Verwendung solcher Katalysatoren führt in der Regel bevorzugt zu Bildung von Monoaminen, wie Methylamin, Ethylamin und/oder iso-Propylamin bei der Verwendung von Ammoniak als Aminierungsmittel. Insbesondere weisen Katalysatorvorläufer auf Basis Ru, beispielsweise als $RuO(OH)_x$, eine hohe Aktivität auf.

Bevorzugt ist auch eine Ausführungsform in der als Katalysator ein Cu-haltiger Katalysator eingesetzt wird. Die Verwendung von Cu-haltigen Katalysatoren führt üblicherweise zu einem höheren Anteil von Piperazinderivaten und/oder Diaminen im Reaktionsaustrag.

Eine weitere bevorzugte Ausführungsform betrifft den Einsatz von Katalysatoren, die Ir enthalten in das erfindungsgemäße Verfahren. Katalysatoren, die Ir enthalten, führen in der Regel zu einem höheren Anteil von Spezialaminen.

Es ist auch möglich die Zusammensetzung des Reaktionsautrags durch die Reaktionstemperatur zu beeinflussen.

So wird beispielsweise bei gleichem Umsatz bei einer niedrigeren Reaktionstemperatur in der Regel die Bildung von Spezialaminen und acyclischen Aminen, wie Diamine und Alkanolamine, begünstigt.

Wird die Reaktion bei einer höheren Reaktionstemperatur bis zum selben Umsatz durchgeführt, so werden im Allgemeinen Piperazin oder Piperazinderivaten durch Zyklisierungsreaktionen gebildet. Bei einer höheren Reaktionstemperatur nehmen bei gleichem Umsatz in der Regel Deaminierungsreaktionen zu, beispielsweise werden aus Aminomethylpiperazin Piperazin gebildet oder aus Ethylendiamin wird Monoethylamin gebildet.

Die Zusammensetzung des Reaktionsaustrags kann weiterhin durch den Zuckeralkohol-Umsatz beeinflusst werden.

So ist zu beobachten, dass bei hohen Umsätzen, unabhängig vom eingesetzten Katalysator, in der Regel die Anzahl an Funktionalitäten abnimmt (Defunktionalisierung), d.h. es werden beispielsweise Di- oder Monoamine aus Triaminen gebildet oder Methylpiperazine aus Aminomethylpiperazinen.

Weiterhin wird bei hohen Zuckeralkohol-Umsätzen eine Zunahme der Zyklisierung und damit einhergehend die Bildung von Piperazin bzw. Piperazinderivaten beobachtet

Hohe Zuckeralkohol-Umsätze, beispielsweise Zuckeralkohol-Umsätze von 80% und mehr, bevorzugt 90% und mehr, besonders bevorzugt 99% und mehr, begünstigen in der Regel die Bildung von Reaktionsausträgen mit einem hohen Anteil an zyklischen Aminen, wie Piperazin und/oder Piperazinderivaten.

Mittlere Zuckeralkohol-Umsätze, beispielsweise Zuckeralkohol-Umsätze von 30 bis 80%, vorzugsweise 40 bis 70% und besonders bevorzugt 50 bis 60%, begünstigen in der Regel die Bildung von Spezialaminen.

Der Zuckeralkohol-Umsatz kann durch eine Reihe von Verfahrensparametern, wie Druck, Temperatur, das molare Verhältnis von Aminierungsmittel, insbesondere Ammoniak, zu Zuckeralkohol, sowie die Reaktions- bzw. Verweilzeit beeinflusst werden.

Der Zuckeralkohol-Umsatz ($U_{Zuckeraikohol}$) ist für den Fachmann durch gaschromatographische Analyse routinemäßig feststellbar und wird üblicherweise wie folgt angegeben:

$$U_{Zuckeralkohol} = (F\%_{Zuckeralkohol, Anfang} - F_{Zuckeralkohol, Ende})/ F_{Zuckeralkohol, Anfang};$$

wobei $F\%_{Zuckeralkohol, Anfang}$ und $F\%_{Zuckeralkohol, Ende}$, die mittels Gaschromatographie ermittelten Flächenprozente unterhalb des Zuckeralkohol-Signals darstellen, das am Anfang bzw. Ende der Reaktion bzw. am Eingang bzw. Ausgang des Reaktor gemessen wurde.

Hohe Zuckeralkohol-Umsätze, beispielsweise 80 bis 100%, können beispielsweise durch eine Erhöhung der Temperatur oder einer Erhöhung des molaren Verhältnisses von Aminierungsmittel, insbesondere Ammoniak, zu Zuckeralkohol erreicht werden. Beispielsweise können hohe Zuckeralkohol-Umsätze in einem Temperaturbereich von in der Regel 200 bis 400°C, bevorzugt 220 bis 350°C, erzielt werden.

Üblicherweise beträgt das molare Verhältnis von Aminierungsmittel, insbesondere von Ammoniak, zu Zuckeralkohol zur Erzielung von hohen Zuckeralkohol-Umsätzen im Bereich von 5:1 bis 250:1, vorzugsweise 10:1 bis 150:1.

Bei einem kontinuierlichen Verfahren ist es möglich einen höheren Zuckeralkohol-Umsatz durch eine Verringerung der Katalysatorbelastung zu bewirken.

Hohe Zuckeralkohol-Umsätze werden in der Regel bei Katalysatorbelastungen im Bereich von 0,05 bis 0,6 kg Zuckeralkohol pro Liter Katalysator (Schüttvolumen) und Stunde, bevorzugt 0,05 bis 0,2 pro Liter Katalysator (Schüttvolumen) und Stunde erzielt. Weiterhin ist es möglich einen hohen Zuckeralkohol-Umsatz bei einem diskontinuierlichen Verfahren durch eine Erhöhung der Verweilzeit oder durch eine Erhöhung der Katalysatorkonzentration zu bewirken. Üblicherweise werden hohe Zuckeralkohol-Umsätze bei Verweilzeiten von 16 bis 72 Stunden, bevorzugt 20 bis 48 Stunden und besonders bevorzugt 24 bis 32 Stunden erzielt, wobei die Verweilzeit in Abhängigkeit der Katalysatorkonzentration auch kürzer oder länger ausfallen kann, um einen hohen Zuckeralkohol-Umsatz zu erzielen.

Mittlere Zuckeralkohol-Umsätze, beispielsweise 30 bis 80%, können beispielsweise durch eine Verringerung der Temperatur oder einer Verringerung des molaren Verhältnisses von Ammoniak zu Zuckeralkohol erreicht werden.

Mittlere Zuckeralkohol-Umsätze können beispielsweise in einem Temperaturbereich von in der Regel 150 bis 300°C, bevorzugt von 150 bis 220°C, erzielt werden. Üblicherweise liegt das molare Verhältnis von Aminierungsmittel, insbesondere Ammoniak, zu Zuckeralkohol bei der Erzielung von hohen Umsätzen im Bereich von 1:1 bis 100:1, vorzugsweise 2,5:1 bis 50:1.

Es ist möglich eine Verringerung des Zuckeralkohol-Umsatzes bei einem kontinuierlichen Verfahren durch eine Erhöhung der Katalysatorbelastung zu bewirken. Mittlere Zuckeralkohol-Umsätze werden in der Regel bei Katalysatorbelastungen im Bereich von 0,1 bis 1,2 kg Zuckeralkohol pro Liter Katalysator (Schüttvolumen) und Stunde, bevorzugt 0,2 bis 0,6 pro Liter Katalysator (Schüttvolumen) und Stunde erzielt.

Bei einem diskontinuierlichen Verfahren ist des möglich den Zuckeralkohol-Umsatz durch eine Verkürzung der Verweilzeit oder durch eine Reduzierung der Katalysatorkonzentration zu verringern. Mittlere Zuckeralkohol-Umsätze werden in der Regel bei Verweilzeiten von 5 bis 20 Stunden, vorzugsweise 10 bis 16 Stunden, erzielt, wobei die Verweilzeit in Abhängigkeit der Katalysatorkonzentration auch kürzer oder länger ausfallen kann, um einen mittleren Zuckeralkohol-Umsatz zu erzielen.

In einer besonders bevorzugten Ausführungsform wird ein Katalysator enthaltend Ni und/oder Co verwendet und ein mittlerer Zuckeralkohol-Umsatz, beispielsweise ein Zuckeralkohol-Umsatz von 30 bis 80%, vorzugsweise 40 bis 70% und besonders bevorzugt 50 bis 60%, eingestellt.

Ein mittlerer Zuckeralkohol-Umsatz kann üblicherweise wie voranstehend beschrieben eingestellt werden.

Bei einem mittleren Zuckeralkohol-Umsatz und der Verwendung von nickelhaltigen und/oder cobalthaltigen Katalysatoren, wird in der Regel bevorzugt ein hoher Anteil an Spezialaminen gebildet.

Der durch diese besondere Ausführungsform des Verfahrens erhaltene Reaktionsaustrag enthält einen Anteil von Spezialaminen von in der Regel mehr als 5 Gew.-%, vorzugsweise mehr als 10 Gew.-%, bezogen auf die Gesamtmasse des gebildeten Amine.

In einer weiteren bevorzugten Ausführungsform wird ein Ir-haltiger Katalysator verwendet und ein mittlerer Zuckeralkohol-Umsatz eingestellt.

Die Einstellung eines mittleren Zuckeralkohol-Umsatzes, beispielsweise 30 bis 80%, kann im Allgemeinen in der voranstehend aufgeführten Weise erzielt werden.

Der durch diese besondere Ausführungsform des Verfahrens erhaltene Reaktionsaustrag enthält einen Anteil von Spezialaminen von in der Regel mehr als 5 Gew.-%, vorzugsweise mehr als 10 Gew.-%, bezogen auf die Gesamtmasse der gebildeten Amine

In einer weiteren bevorzugten Ausführungsform wird bei Verwendung eines Katalysators enthaltend Ni und/oder Co ein hoher Zuckeralkohol-Umsatz, beispielsweise mehr als 80%, bevorzugt mehr als 90%, besonders bevorzugt mehr als 99%, eingestellt. Hohe Zuckeralkohol-Umsätze können beispielsweise wie voranstehend beschrieben eingestellt werden.

Der durch diese besondere Ausführungsform des Verfahrens erhaltene Reaktionsaustrag enthält einen Anteil von Piperazin und/oder Piperazinderivaten von in der Regel mehr als 10 Gew.-%, vorzugsweise 20 Gew.-% bis 80 Gew.-% und besonders bevorzugt 30 bis 70 Gew.-%, bezogen auf die Gesamtmasse der gebildeten Amine.

In einer weiteren bevorzugten Ausführungsform wird ein Katalysator, der ein Metall oder mehrere Metalle der 5. Periode der Gruppen 8 und/oder 9 und/oder 10 und/oder 11 enthält, verwendet und ein hoher Zuckeralkohol-Umsatz eingestellt.

Die Einstellung eines hohen Zuckeralkohol-Umsatzes, beispielsweise mehr als 80%, kann im Allgemeinen in der voranstehend aufgeführten Weise erzielt werden.

Das durch diese besondere Ausführungsform des Verfahrens erhaltene Reaktionsaustrag enthält einen Anteil von Monoaminen von in der Regel mehr als 10 Gew.-%, bezogen auf die Gesamtmasse der gebildeten Amine.

In einer ganz besonders bevorzugten Ausführungsform wird ein Ni- und/oder Cohaltiger Katalysator in einem Temperaturbereich von 150 bis 220°C verwendet. In dieser bevorzugten Ausführungsform wird ein hoher Zuckeralkohol-Umsatz, beispielsweise mehr als 80%, bevorzugt mehr als 90%, besonders bevorzugt mehr als 99%, eingestellt, beispielsweise in dem man die Katalysatorbelastung verringert oder die Verweilzeit erhöht. Die ganz besondere Ausführungsform unterscheidet sich von der voranstehend genannten Ausführungsform unter Verwendung von Ni- und/oder Co-haltigen Katalysatoren bei hohem Zuckeralkohol-Umsatz dadurch, dass ein hoher Zuckeralkohol-Umsatz bei Temperaturen im Bereich von 150 und 220°C anstelle von 220 bis 350°C eingestellt wird.

In der kontinuierlichen Variante dieser ganz besonderen Ausführungsform beträgt die Katalysatorbelastung in der Regel im Bereich von 0,05 bis 0,6 kg Zuckeralkohol pro Liter Katalysator (Schüttvolumen) und Stunde, bevorzugt 0,05 bis 0,2 pro Liter Katalysator (Schüttvolumen) und Stunde.

In der diskontinuierlichen Variante dieser ganz besonderen Ausführungsform kann ein hoher Zuckeralkohol-Umsatz im Allgemeinen durch eine Verlängerung der Verweilzeit oder durch eine Erhöhung der Katalysatorkonzentration erzielt werden. Üblicherweise beträgt die Verweilzeit in dieser besonders bevorzugten Ausführungsform bei einer Verweilzeit mehr als 20 Stunden, bevorzugt mehr als 24 Stunden und besonders bevorzugt mehr als 30 Stunden, wobei die Verweilzeit in Abhängigkeit von der Katalysatorkonzentration auch kürzer bzw. länger betragen kann.

Üblicherweise liegt das molare Verhältnis von Aminierungsmittel, insbesondere von Ammoniak, zu Zuckeralkohol zur Erzielung von hohen Zuckeralkohol-Umsätzen in dieser ganz besonderen Ausführungsform im Bereich von 5:1 bis 250:1, vorzugsweise 10:1 bis 150:1.

Der durch diese ganz besondere Ausführungsform des Verfahrens erhaltene Reaktionsaustrag enthält einen Anteil von Diaminopropan, Diaminopropanol und Triaminopropan von mehr als 10 Gew.-%, vorzugsweise 15 Gew.-% bis 80 Gew.-% und besonders bevorzugt 20 bis 70 Gew.-%, bezogen auf die Gesamtmasse des Amine.

Der Reaktionsaustrag enthält in der Regel die erfindungsgemäß hergestellten Amine, sowie Wasser, Aminierungsmittel, Wasserstoff und ggf. nicht umgesetzten Zuckeralkohol.

Aus dem Reaktionsaustrag werden, nachdem dieser zweckmäßigerweise entspannt worden ist, das überschüssige Aminierungsmittel und der Wasserstoff entfernt. Das überschüssige Aminierungsmittel und der Wasserstoff werden vorteilhaft wieder in die Reaktionszone zurückgeführt.

Nach Abtrennung des Aminierungsmittels und des Wasserstoffs wird der so erhaltene Reaktionsaustrag in der Regel aufgearbeitet.

In der Regel wird der Reaktionsaustrag entwässert, da Wasser und Amine Azeotrope bilden können, die die destillative Trennung der einzelnen Amine des Reaktionsaustrages erschweren können.

Die Entwässerung des wasserhaltigen Reaktionsaustrags erfolgt üblicherweise durch Inkontaktbringen des wässrigen Reaktionsaustrags mit Natronlauge.

Die Konzentration der Natronlauge beträgt üblicherweise 20 bis 80%, bevorzugt 30 bis 70% und besonders bevorzugt 40 bis 60%.

Das Volumenverhältnis von zugegebener Natronlauge und dem Reaktionsaustrag beträgt üblicherweise zwischen 0,5:1 bis 2:1, vorzugsweise 1:1.

Das Inkontaktbringen des Reaktionsaustrages mit Natronlauge kann durch Zuführen der Natronlauge in dem Reaktionsreaktor erfolgen, in dem zuvor die hydrierende Aminierung von Zuckeralkohol durchgeführt wurde. Bei kontinuierlicher Reaktionsführung kann die Natronlauge als kontinuierlicher Strom am Reaktorausgang zudosiert werden. Sie kann aber auch in einer Destillationskolonne im Gegenstrom mit dem dampfförmigen Reaktionsaustrag in Sinne einer Extraktivdestillation in Kontakt gebracht werden. Verfahren zur Extraktivdestillation sind besipielsweise in GB-A-1,1,02,370 oder EP-A- 1312600 beschrieben.

In einer bevorzugten Variante wird der Reaktionsaustrag entwässert, beispielsweise wenn ein mittlerer Zuckeralkohol-Umsatz eingestellt wird, da sich Zuckeralkohol üblicherweise zusammen mit der wässrigen Phase von den gebildeten Aminen in der Regel vollständig bzw. zumindest nahezu vollständig abtrennen lässt.

Die Auftrennung des Reaktionsaustrags kann durch Destillation bzw. Rektifikation, Flüssigextraktion oder Kristallisation erfolgen, wobei die Auftrennung in einer oder mehreren Stufen erfolgen kann, wobei die Anzahl der Stufen in der Regel abhängig ist von der Anzahl der im Reaktionsaustrag vorhandenen Komponenten.

Der Reaktionsaustrag kann in Fraktionen aufgetrennt werden, die ein Gemisch verschiedener Aminkomponenten enthalten, oder in Fraktionen, die nur eine Aminkomponente enthalten.

Beispielsweise kann zuerst eine Trennung in Fraktionen erfolgen, die mehr als eine Aminkomponente enthalten. Diese Fraktionen können nachfolgend, beispielsweise durch eine Feindestillation, in die einzelnen Verbindungen bzw. Komponenten aufgetrennt werden.

Nicht umgesetzter Zuckeralkohol kann in das Verfahren zurückgeführt werden.

Die bei der Aufarbeitung des Reaktionsaustrags erhaltenen Fraktionen, enthaltend ein oder mehrere Amine, können beispielsweise als Additive bei der Beton und-oder Zementherstellung verwendet werden.

Solche Fraktionen enthalten beispielsweise:

0 bis 5 Gew.-% Diamine, wie 1,2-Diaminopropan;
5 bis 20 Gew.-% Piperazinderivate, wie 2-Methylpiperazin, 2,5-Bis-(Aminomethyl)-piperazin, 3,5-Bis-(Aminomethyl)-piperazin, 2-Aminomethyl-6-methyl-piperazin, 3-Aminomethyl-5-methyl-piperazin und/oder 3-Aminomethyl-6-methyl-piperazin;

10 bis 30 Gew.-% z Spezialamine, wie 1,2,3-Triaminopropan, 1,2-Diamino-propan-3-ol und/oder 1,3-Diamino-propan-2-ol und

20 bis 45 Gew.-% Zuckeralkohol.

Weiterhin kann eine solche Fraktion 15 bis 30 Gew.-% Wasser und weitere Komponenten, wie Monoamine, Diamine, Piperazin, Piperazinderivate und/oder Alkanolamine enthalten.

Die erfindungsgemäß erhaltenen Amine, wie Monoamine ausgewählt aus der Gruppe bestehend aus Methylamin, Ethylamin, i-Propylamin und n-Propylamin, und/oder ein oder mehrere Diamine ausgewählt aus der Gruppe bestehend aus Ethylendiamin, 1,2-Propandiamin und 1,3-Propandiamin, 1,2-Butandiamin, 1,3-Butandiamin, 1,4-Butandiamin und/oder

ein oder mehrere Alkanolamine, ausgewählt aus der Gruppe bestehend aus Monoethanolamin, 2-Aminopropan-1-ol und 1-Aminopropan-2-ol, und/oder ein oder mehrerer zuckeralkoholanaloge Spezialamine, ausgewählt aus der Gruppe bestehend aus 1,2,3-Triaminopropan, 1,3-Diaminopropan-2-ol, 1,2-Diaminopropan-3-ol, 1-Aminopropandiol, 2-Aminopropandiol, Glucosamin und Isomaltin und/oder Piperazin, und/oder

ein oder mehrere Piperazinderivate, ausgewählt aus der Gruppe bestehend aus aus aus 2-Methylpiperazin, 2,6-Dimethylpiperazin, 2,5-Dimethylpiperazin, 2,5-Bis(Aminomethyl)-piperazin, 2,6-Bis(Aminomethyl)-piperazin, 2-Aminomethyl-5-methyl-piperazin und 2-Aminomethyl-6-methyl-piperazin,

können als Synthesebaustein für die Herstellung von Tensiden, Arznei- und Pflanzenschutzmitteln, Stabilisatoren, Lichtschutzmitteln, Polymeren, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren verwendet werden.

Die Vorteile der vorliegenden Erfindung bestehen darin, dass Zuckeralkohol als Quelle für die Herstellung von Aminen effektiv und wirkungsvoll genutzt wird. Es wird ein Verfahren zur Verfügung gestellt, dass es sowohl erlaubt wichtige technische Amine zu erhalten, als auch Spezialamine sowie Piperazinderivate, um den Rohstoff Zuckeralkohol optimal zu verwerten.

Die Umsetzung von Zuckeralkohol beinhaltet nur wenige Reaktionsschritte.

Durch einfach durchzuführende Einstellungen der Verfahrensbedingungen, wie beispielsweise Druck und Temperatur, sowie durch die Wahl des Katalysators, kann die Zusammensetzung des Reaktionsaustrags innerhalb gewisser Grenzen reguliert werden, um auf Nachfrage- und Absatzschwankungen flexibel reagieren zu können.

Durch dieses Verfahren können wichtige technische Amine erhalten werden, beispielsweise Monoamine, wie Methyl-amin, Ethylamin, i-Propylamin oder n-Propylamin, Diamine, wie Ethylendiamin, 1,2-Propandiamin oder 1,3-Propandiamin, Alkanolamine, wie Monoethanolamin, 2-Aminopropan-1-ol oder 1-Aminopropan-2-ol, oder Piperazin, die bislang aus petrochemischen Ausgangsstoffen hergestellt wurden. Es werden aber auch neue Spezialamine erhalten. Diese Verbindungen weisen eine hohe Anzahl von Funktionalitäten aus und können daher wichtige Zwischenprodukte in der Synthese von organischen Verbindungen, wie Pflanzenschutzmitteln, Pharmazeutika, Stabilisatoren etc. darstellen.

Außerdem werden Derivate von Piperazin (Piperazinderivate), wie 2-Methylpiperazin, 2,6-Dimethylpiperazin, 2,5-Dimethylpiperazin, 2,5-Bis-(Aminomethyl)-piperazin, 2,6-Bis-(Aminomethyl)-piperazin, 2-Aminomethyl-5-methyl-piperazin oder 2-Aminomethyl-6-methyl-piperazin, erhalten, die ebenfalls wichtige Synthesebausteine darstellen können.

Die Tendenz zur Bildung von aromatischen Nebenprodukten, insbesondere an Pyrazinen und Pyrazinderivaten, ist beim erfindungsgemäßen Verfahren niedrig.

Das erfindungsgemäße Verfahren wird anhand von nachfolgenden Beispielen erläutert.

Beispiele 1 bis 2 sowie Vergleichsbeispiele 1 bis 3:

Allgemeine Arbeitsvorschrift:

In einen Hochdruckautoklaven wurden 5 g pulverförmiger Katalysator, 15 g Substrat gelöst in 22,5 g Wasser gegeben. Der Reaktor wurde inertisiert und 90 g Ammoniak zugegeben. Das Inertgas wurde gegen Wasserstoff ausgetauscht und anschließend ein Wasserstoffdruck von 20 bar aufgepresst. Der Reaktor wurde auf 100°C aufgeheizt und der Wasserstoffdruck nach Erreichen der Temperatur auf 50 bar erhöht. Die Reaktionsmischung wurde bei 100°C und 50 bar 1 Stunde gerührt. Anschließend wurde die Temperatur kontinuierlich innerhalb von 2 Stunden auf 200°C erhöht und die Reaktionsmischung nach Erreichen der 200°C 1 Stunde gerührt. Anschließend wurde der Druck durch Aufpressen von Wasserstoff auf 200 bar erhöht. Der Druck wurde während der Gesamtdauer der Reaktion konstant bei diesem Wert gehalten. Nach einer Reaktionszeit von 32 Stunden wurde eine Probe entnommen, die analysiert wurde. Die Gehalte der Verbindungen wurden mittels Gaschromatographie (Bedingungen: Kapillarsäule RTX 5 Amin 30m, Filmdicke 1,5 Mikrometer, Durchmesser 0,32 mm, Methode: 5 min. 60°C, dann mit 7°C/min aufheizen auf 280°C und bei 280°C 20 min. ausheizen) als Flächenprozente (F%) ermittelt. Hierbei beziehen sich die Flächenprozente der Signale auf die Gesamtfläche unterhalb der gemessenen Signale mit Ausnahme des Wassersignals.

Die angegebenen Zuckeralkohol-Umsätze beziehen sich auf die ermittelten Flächenprozente vor Beginn und am Ende der Reaktion.

Beispiel 1:

Die Herstellung erfolgte, wie in der allgemeinen Arbeitsvorschrift beschrieben. Als Substrat wurde Sorbitol eingesetzt. Als Katalysator wurde ein Nickel-Katalysator nach Raney verwendet. Die Endtemperatur betrug 200°C.

Die gaschromatographische Analyse nach 32 Stunden ergab folgende Zusammensetzung:

Ethylendiamin: 8%; 1,2-Propylendiamin: 12%; Piperazin: 4%; 2-Methylpiperazin: 12%; 2,6-Dimethyl-piperazin: 0%; 2,5-Dimethyl-piperazin: 4%; Heteroaromaten: 6%.

Der Sorbitol-Umsatz betrug ca. 100%.

Beispiel 2:

Die Herstellung erfolgte, wie in der allgemeinen Arbeitsvorschrift beschrieben. Als Substrat wurde Sorbitol verwendet. Als Katalysator wurde ein Katalysator eingesetzt, der durch Vorreduktion aus einem Katalysatorvorläufer erhalten wurde, dessen katalytisch aktive Masse vor der Reduktion mit Wasserstoff 13 Gew.-% Cu, berechnet als CuO, 28 Gew.-% Ni, berechnet als NiO, 28 Gew.-% Co, berechnet als CoO und 31 Gew.-% Zr, berechnet als $ZrO_2$, enthielt. Die Endtemperatur betrug 200°C.

Die gaschromatographische Analyse nach 32 Stunden ergab folgende Zusammensetzung:

Ethylendiamin: 8%; 1,2-Propylendiamin: 17%; Piperazin: 11%; 2-Methylpiperazin: 25%; 2,6-Dimethyl-piperazin: 8%; 2,5-Dimethyl-piperazin: 6%; Heteroaromaten: 0%. Der Sorbitol-Umsatz betrug ca. 100%.

Vergleichsbeispiel 1:

Die Herstellung erfolgte, wie in der allgemeinen Arbeitsvorschrift beschrieben. Als Substrat wurde Glucose eingesetzt. Als Katalysator wurde ein Nickel-Katalysator nach Raney verwendet. Die Endtemperatur betrug 200°C.

Die gaschromatographische Analyse nach 32 Stunden ergab folgende Zusammensetzung:

Ethylendiamin: 15%; 1,2-Propylendiamin: 13%; Piperazin: 3%; 2-Methylpiperazin: 12%; 2,6-Dimethyl-piperazin: 1%; 2,5-Dimethyl-piperazin: 7%; Heteroaromaten: 11%. Der Glucose-Umsatz betrug ca. 100%.

Vergleichsbeispiel 2:

Die Herstellung erfolgte, wie in der allgemeinen Arbeitsvorschrift beschrieben. Als Substrat wurde Glucose verwendet. Als Katalysator wurde ein Katalysator eingesetzt, der durch Vorreduktion aus einem Katalysatorvorläufer erhalten wurde, dessen katalytisch aktive Masse vor der Reduktion mit Wasserstoff 13 Gew.-% Cu, berechnet als CuO, 28 Gew.-% Ni, berechnet als NiO, 28 Gew.-% Co, berechnet als CoO und 31 Gew.-% Zr, berechnet als $ZrO_2$, enthielt. Die Endtemperatur betrug 200°C.

Die gaschromatographische Analyse nach 32 Stunden ergab folgende Zusammensetzung:

Ethylendiamin: 7%; 1,2-Propylendiamin: 15%; Piperazin: 3%; 2-Methylpiperazin: 9%; 2,6-Dimethyl-piperazin: 0%; 2,5-Dimethyl-piperazin: 1%; Heteroaromaten: 14%. Der Glucose-Umsatz betrug ca. 100%.

Vergleichsbeispiel 3:

Die Herstellung erfolgte, wie in der allgemeinen Arbeitsvorschrift beschrieben. Als Substrat wurde Saccharose eingesetzt. Als Katalysator wurde ein Nickel-Katalysator nach Raney verwendet. Die Endtemperatur betrug 200°C.

Die gaschromatographische Analyse nach 32 Stunden ergab folgende Zusammensetzung:

Ethylendiamin: 6%; 1,2-Propylendiamin: 5%; Piperazin: 8%; 2-Methylpiperazin: 20%; 2,6-Dimethyl-piperazin: 2%; 2,5-Dimethyl-piperazin: 0%; Heteroaromaten: 21%. Der Saccharose-Umsatz betrug ca. 100%.

Beispiel 3:

In einen Hochdruckautoklaven wurden 21 g Katalysator 15 g Sorbitol gelöst in 61,5 g Wasser und 0,75 g Calciumoxid gegeben. Als Katalysator wurde ein Gemisch aus einem auf Aktivkohle geträgerter Palladiumkatalysator mit einem Pd-Gehalt von ca. 5 Gew.-% und Wasser eingesetzt, wobei der Wassergehalt des Gemisches 64% betrug.

Der Reaktor wurde inertisiert. Das Inertgas wurde gegen Wasserstoff ausgetauscht und anschließend ein Wasserstoffdruck von 100 bar aufgepresst. Der Reaktor wurde auf eine Endtemperatur von 230°C aufgeheizt und der Wasserstoffdruck auf 250 bar erhöht. Nach 10 Stunden wurde der Reaktor auf Raumtemperatur abgekühlt und langsam entspannt.

Der Reaktorinhalt wurde vom Katalysator abfiltriert und erneut in einen Hochdruckautoklaven gegeben. Es wurden 5 g reduzierter Katalysator hinzugefügt. Als Katalysator wurde ein Katalysator eingesetzt, der durch Vorreduktion aus einem Katalysatorvorläufer erhalten wurde, dessen katalytisch aktive Masse vor der Reduktion mit Wasserstoff 13 Gew.-% Cu, berechnet als CuO, 28 Gew.-% Ni, berechnet als NiO, 28 Gew.-% Co, berechnet als CoO und 31 Gew.-% Zr, berechnet als $ZrO_2$, enthielt. Der Reaktor wurde inertisiert und es wurden 55 g Ammoniak hinzugegeben und ein Druck von 20 bar Wasserstoff aufgepresst. Der Reaktor wurde auf 100°C aufgeheizt und der Wasserstoffdruck nach Erreichen der Temperatur auf 50 bar erhöht. Die Reaktionsmischung wurde bei 100°C und 50 bar 1 Stunde gerührt. Anschließend wurde die Temperatur kontinuierlich innerhalb von 2 Stunden auf 190°C erhöht und die Reaktionsmischung nach Erreichen der 190°C 1 Stunde gerührt. Anschließend wurde der Druck durch Aufpressen von Wasserstoff auf 200 bar erhöht. Der Druck wurde während der Gesamtdauer der Reaktion konstant bei diesem Wert gehalten. Nach einer Reaktionszeit von 32 Stunden wurde eine Probe entnommen, die analysiert wurde. Die Gehalte der Verbindungen wurden mittels Gaschromatographie (Bedingungen: Kapillarsäule RTX 5 Amin 30m, Filmdicke 1,5 Mikrometer, Durchmesser 0,32 mm, Methode: 5 min. 60°C, dann mit 7°C/min aufheizen auf 280°C und bei 280°C 20 min. ausheizen) als Flächenprozente (F%) ermittelt. Hierbei beziehen sich die Flächenprozente der Signale auf die Gesamtfläche unterhalb der gemessenen Signale mit Ausnahme des Wassersignals. Die angegebenen Zuckeralkohol-Umsätze beziehen sich auf die ermittelten Flächenprozente vor Beginn und am Ende der Reaktion.

Die gaschromatographische Analyse nach 32 Stunden ergab folgende Zusammensetzung: Ethylendiamin: 9%; 1,2-Propylendiamin: 28%; Piperazin: 6%; 2-Methylpiperazin: 17%; 2,6-Dimethyl-piperazin: 11%; 2,5-Dimethyl-piperazin: 3%; Heteroaromaten: 0%. Der Sorbitol-Umsatz betrug ca. 100%.

Beispiel 4:

Die Herstellung erfolgte, wie in der allgemeinen Arbeitsvorschrift beschrieben analog zu Beispiel 1. Es wurde jedoch 75 g Wasser und nur 55 g Ammoniak verwendet. Als Substrat wurde Sorbitol eingesetzt. Als Katalysator wurde ein Katalysator eingesetzt, der durch Vorreduktion aus einem Katalysatorvorläufer erhalten wurde, dessen katalytisch aktive Masse vor der Reduktion mit Wasserstoff 13 Gew.-% Cu, berechnet als CuO, 28 Gew.-% Ni, berechnet als NiO, 28 Gew.-% Co, berechnet als CoO und 31 Gew.-% Zr, berechnet als $ZrO_2$, enthielt. Die Endtemperatur betrug 190°C.

Die gaschromatographische Analyse nach 32 Stunden ergab folgende Zusammensetzung: Ethylendiamin: 6%; 1,2-Propylendiamin: 13%; Piperazin: 4%; 2-Methylpiperazin: 18%; 2,6-Dimethyl-piperazin: 12%; 2,5-Dimethyl-piperazin: 4%; Heteroaromaten: 0%. Der Sorbitol-Umsatz betrug ca. 100%.

Beispiele 5 bis 10:

Allgemeiner Arbeitsvorschrift:

In einem Hochdruckautoklaven wurden 0,5 g pulverförmiger Katalysator, 4,8 g Sorbitol und 6,5 g Wasser gegeben. Der Reaktor wurde inertisiert und Ammoniak zugegeben. Das Inertgas wurde gegen Wasserstoff ausgetauscht und anschließend ein Wasserstoffdruck von 50 bar aufgepresst. Unter Rühren wurde der Reaktor innerhalb von 2 Stunden auf 200°C aufgeheizt und nach Erreichen dieser Temperatur der Druck durch Aufpressen von Wasserstoff auf 200 bar erhöht. Der Druck wurde während der Gesamtdauer der Reaktion konstant bei diesem Wert gehalten. Nach einer Reaktionszeit von 24 Stunden wurde der Reaktor auf RT abgekühlt und bei Raumtemperatur langsam entspannt. Der entgaste Reaktorinhalt wurde analysiert. Die Gehalte der Verbindungen mittels Gaschromatographie (Bedingungen: Kapillarsäule RTX 5 Amin 30m, Filmdicke 1,5 Mikrometer, Durchmesser 0,32 mm, Methode: 5 min. 60°C, dann mit 7°C/min aufheizen auf 280°C und bei 280°C 20 min. ausheizen) als Flächenprozente (F%) ermittelt. Hierbei beziehen sich die Flächenprozente der Signale auf die Gesamtfläche unterhalb der gemessenen Signale mit Ausnahme des Wassersignals.

Die angegebenen Zuckeralkohol-Umsätze beziehen sich auf die ermittelten Flächenprozente vor Beginn und am Ende der Reaktion.

Beispiel 5:

Die Herstellung erfolgte, wie in der allgemeinen Arbeitsvorschrift beschrieben. Als Katalysator wurde ein Katalysator eingesetzt, der durch Vorreduktion aus einem Katalysatorvorläufer erhalten wurde, dessen katalytisch aktive Masse vor der Reduktion mit Wasserstoff 13 Gew.-% Cu, berechnet als CuO, 28 Gew.-% Ni, berechnet als NiO, 28 Gew.-% Co, berechnet als CoO und 31 Gew.-% Zr, berechnet als $ZrO_2$, enthielt. Die Vorreduktion des Katalysatorvorläufers erfolgte 20 Stunden bei einer Temperatur von 280°C unter einer reinen Wasserstoff-Atmosphäre.

Die gaschromatographische Analyse ergab folgende Zusammensetzung:

Ethylendiamin: 1%; Monoethanolamin: 1%, Monoethylenglykol: 2%, 1,2-Propandiamin: 2%, 2-Aminopropan-1-ol: 4; 2-Methylpiperazin: 2%; 2,6-Dimethylpiperazin: 3%; 2,5-Dimethyl-piperazin: 3%; $C_4$-Diamine (1,2-Butandiamin, 1,3-Butandiamin, 1,4-Butandiamin): 3%; Heteroaromaten: 0%; Sorbitol: 36%

Der Sorbitol-Umsatz betrug ca. 64%.

Beispiel 6:

Die Herstellung erfolgte, wie in der allgemeinen Arbeitsvorschrift beschrieben. Als Katalysator wurde ein Katalysator eingesetzt, der durch Vorreduktion aus einem Katalysatorvorläufer erhalten wurde, dessen katalytisch aktive Masse aus $LiCoO_2$ bestand. Die Vorreduktion des Katalysatorvorläufers erfolgte 20 Stunden bei einer Temperatur von 300°C unter einer reinen Wasserstoff-Atmosphäre.

Die gaschromatographische Analyse ergab folgende Zusammensetzung:

Ethylendiamin: 0%; Monoethanolamin: 4%, Monoethylenglykol: 0%, 1,2-Propandiamin: 0%, 2-Aminopropan-1-ol: 5%; 2-Methylpiperazin: 3%; 2,6-Dimethylpiperazin: 3%;
2,5-Dimethyl-piperazin: 2%; $C_4$-Diamine (1,2-Butandiamin, 1,3-Butandiamin, 1,4-Butandiamin): 8%; Heteroaromaten: 1%; Sorbitol: 22%

Der Sorbitol-Umsatz betrug ca. 78%.

Beispiel 7:

Die Herstellung erfolgte, wie in der allgemeinen Arbeitsvorschrift beschrieben. Als Katalysator wurde ein Katalysator eingesetzt, der durch Vorreduktion aus einem Katalysatorvorläufer erhalten wurde, dessen katalytisch aktive Masse aus $RuO(OH)_x$ bestand.

Die gaschromatographische Analyse ergab folgende Zusammensetzung:

Ethylamin: 2%; Propylamin: 2%; Ethylendiamin: 0%; Monoethanolamin: 0%, Monoethylenglykol: 1%, 1,2-Propandiamin: 0%, 2-Aminopropan-1-ol: 0%; 2-Methylpiperazin: 0%; 2,6-Dimethylpiperazin: 0%; 2,5-Dimethyl-piperazin: 2%; $C_4$-Diamine (1,2-Butandiamin, 1,3-Butandiamin, 1,4-Butandiamin): 0%; Heteroaromaten: 0%; Sorbitol: 13%

Der Sorbitol-Umsatz betrug ca. 87%.

Beispiel 8:

Die Herstellung erfolgte, wie in der allgemeinen Arbeitsvorschrift beschrieben. Als Katalysator wurde ein Katalysator eingesetzt, der durch Vorreduktion aus einem Katalysatorvorläufer erhalten wurde, dessen katalytisch aktive Masse vor der Reduktion mit Wasserstoff 85 Gew.-% Co, berechnet als CoO, und 5 Gew.-% Mn, berechnet als $Mn_3O_4$, enthielt.

Die Vorreduktion des Katalysatorvorläufers erfolgte 12 Stunden bei einer Temperatur von 280°C unter einer reinen Wasserstoff-Atmosphäre.

Die gaschromatographische Analyse ergab folgende Zusammensetzung:

Ethylendiamin: 1%; Monoethanolamin: 0%, Monoethylenglykol: 0%, 1,2-Propandiamin: 0%, 2-Aminopropan-1-ol: 1%; 2-Methylpiperazin: 0%; 2,6-Dimethylpiperazin: 0%;
2,5-Dimethyl-piperazin: 0%; $C_4$-Diamine (1,2-Butandiamin, 1,3-Butandiamin, 1,4-Butandiamin): 0%; Heteroaromaten: 0%; Sorbitol: 71%

Der Sorbitol-Umsatz betrug ca. 29%.

Beispiel 9:

Die Herstellung erfolgte, wie in der allgemeinen Arbeitsvorschrift beschrieben. Als Katalysator wurde ein Katalysator eingesetzt, der durch Vorreduktion aus einem Katalysatorvorläufer erhalten wurde, dessen katalytisch aktive Masse vor der Reduktion mit Wasserstoff 76 Gew.-% Co, berechnet als CoO, 23 Gew.-% Cu, berechnet als CuO, 9 Gew.-% Mn, berechnet als $MnO_2$, 4 Gew.-% Mo, berechnet als $MoO_3$, 2,7 Gew.-% P, berechnet als $P_2O_5$, und 0,2 Gew.-% Na, berechnet als $Na_2O$, enthielt. Die Vorreduktion des Katalysatorvorläufers erfolgte 20 Stunden bei einer Temperatur von 280°C unter einer reinen Wasserstoff-Atmosphäre.

Die gaschromatographische Analyse ergab folgende Zusammensetzung:
Ethylendiamin: 0%; Monoethanolamin: 0%, Monoethylenglykol: 0%, 1,2-Propandiamin: 1%, 2-Aminopropan-1-ol: 3%; 2-Methylpiperazin: 1%; 2,6-Dimethylpiperazin: 1%; 2,5-Dimethyl-piperazin: 1%; $C_4$-Diamine (1,2-Butandiamin, 1,3-Butandiamin, 1,4-Butandiamin): 2%; Heteroaromaten: 0%; Sorbitol: 58%
Der Sorbitol-Umsatz betrug ca. 42%.
Beispiel 10 (entspricht Beispiel 12 Sorbitol, Raney-Co)
Die Herstellung erfolgte, wie in der allgemeinen Arbeitsvorschrift beschrieben. Als Katalysator wurde Cobalt nach Raney (Raney® 2724 von der Firma Grace Davison) verwendet.
Die gaschromatographische Analyse ergab folgende Zusammensetzung:
Ethylendiamin: 0%; Monoethanolamin: 2%, Monoethylenglykol: 0%, 1,2-Propandiamin: 0%, 2-Aminopropan-1-ol: 2%; 2-Methylpiperazin: 1%; 2,6-Dimethylpiperazin: 0%; 2,5-Dimethyl-piperazin: 1%; $C_4$-Diamine (1,2-Butandiamin, 1,3-Butandiamin, 1,4-Butandiamin): 4%; Heteroaromaten: 0%; Sorbitol: 35%
Der Sorbitol-Umsatz betrug ca. 65%.

## Patentansprüche

1. Verfahren zur Herstellung von Aminen durch Umsetzung von Zuckeralkoholen mit Wasserstoff und einem Aminierungsmittel, ausgewählt aus der Gruppe Ammoniak, Methylamin, Dimethylamin, Ethylamin, Diethylamin, n-Propylamin, Di-n-propylamin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin in Gegenwart eines Katalysators bei einer Temperatur von 100°C bis 400°C und einem Druck von 1 bis 40 Mpa, wobei der Zuckeralkohol Sorbitol ist.

2. Verfahren nach Anspruch 1, **dadurch gekennzeichnet, dass** der Zuckeralkohol durch Hydrierung der entsprechenden Zucker erhalten wurde.

3. Verfahren nach mindestens einem der Ansprüche 1 bis 2, **dadurch gekennzeichnet, dass** der Katalysator ein Metall oder mehrere Metalle oder eine oder mehrere sauerstoffhaltige Verbindungen der Metalle der Gruppen 8 und/oder 9 und/oder 10 und/oder 11 des Periodensystems der Elemente enthält.

4. Verfahren nach Anspruch 3, **dadurch gekennzeichnet, dass** der Katalysator Ni bzw. NiO und/oder Co bzw. CoO enthält.

5. Verfahren nach Anspruch 3 **dadurch gekennzeichnet, dass** der Katalysator ein Schwammkatalysator nach Raney enthaltend Ni und/oder Co ist.

6. Verfahren nach Anspruch 3 **dadurch gekennzeichnet, dass** der Katalysator ein Metall oder mehrere Metalle der 5. Periode der Gruppen 8 und/oder 9 und/oder 10 und/oder 11 des Periodensystems der Elemente enthält.

7. Verfahren nach Anspruch 3 **dadurch gekennzeichnet, dass** der Katalysator Cu bzw. CuO enthält.

8. Verfahren nach Anspruch 3 **dadurch gekennzeichnet, dass** der Katalysator Ir enthält.

9. Verfahren nach mindestens einem der Ansprüche 1 bis 8, **dadurch gekennzeichnet, dass** die Katalysatorbelastung im Bereich von 0,1 bis 1,2 kg Zuckeralkohol pro Liter Katalysator und Stunde und die Temperatur im Bereich von 150 bis 220°C liegt.

10. Verfahren nach mindestens einem der Ansprüche 1 bis 8, **dadurch gekennzeichnet, dass** die Katalysatorbelastung im Bereich von 0,05 bis 0,6 kg Zuckeralkohol pro Liter Katalysator und Stunde und die Temperatur im Bereich von 220 bis 350°C liegt.

11. Verfahren nach Anspruch 10, **dadurch gekennzeichnet, dass** die Temperatur im Bereich von 150 bis 220°C liegt.

12. Verfahren nach mindestens einem der Ansprüche 1 bis 11, **dadurch gekennzeichnet, dass** der Reaktionsaustrag destillativ aufgearbeitet wird.

13. Verfahren nach Anspruch 12 **dadurch gekennzeichnet, dass** der Reaktionsaustrag vor der destillativen Aufarbeitung entwässert wird.

14. Verfahren nach mindestens einem der Ansprüche 1 bis 13, **dadurch gekennzeichnet, dass** das Verfahren in zwei Stufen durchgeführt wird, wobei in der ersten Stufe zunächst die Umsetzung von Zuckeralkoholen mit Wasserstoff in Gegenwart eines Katalysators erfolgt und nachdem ein Teil der Zuckeralkohole umgesetzt wurde in einer zweiten Stufe das Aminierungsmittel zu dem Reaktionsgemisch zugegeben wird.

15. Verfahren nach mindestens einem der Ansprüche 1 bis 14, **dadurch gekennzeichnet, dass** als Aminierungsmittel Ammoniak eingesetzt wird.

**Claims**

1. A process for preparing amines by reacting sugar alcohols with hydrogen and an aminating agent selected from the group of ammonia, methylamine, dimethylamine, ethylamine, diethylamine, n-propylamine, di-n-propylamine, iso-propylamine, diisopropylamine, isopropylethylamine, n-butylamine, di-n-butylamine, s-butylamine, di-s-butylamine, isobutylamine, n-pentylamine, s-pentylamine, isopentylamine, n-hexylamine, s-hexylamine, isohexylamine, cyclohexylamine, aniline, toluidine, piperidine, morpholine, and pyrrolidine in the presence of a catalyst at a temperature of from 100°C to 400°C and a pressure of from 1 to 40 MPa, where the sugar alcohol is sorbitol.

2. The process according to claim 1, wherein the sugar alcohol has been obtained by hydrogenating the corresponding sugars.

3. The process according to at least one of claims 1 and 2, wherein the catalyst comprises one metal or a plurality of metals or one or more oxygen compounds of the metals of groups 8 and/or 9 and/or 10 and/or 11 of the Periodic Table of the Elements.

4. The process according to claim 3, wherein the catalyst comprises Ni or NiO and/or Co or CoO.

5. The process according to claim 3, wherein the catalyst is a Raney sponge catalyst comprising Ni and/or Co.

6. The process according to claim 3, wherein the catalyst comprises one metal or a plurality of metals of the 5th period of groups 8 and/or 9 and/or 10 and/or 11 of the Periodic Table of the Elements.

7. The process according to claim 3, wherein the catalyst comprises Cu or CuO.

8. The process according to claim 3, wherein the catalyst comprises Ir.

9. The process according to at least one of claims 1 to 8, wherein the catalyst hourly space velocity is in the range from 0.1 to 1.2 kg of sugar alcohol per liter of catalyst and hour, and the temperature is in the range from 150 to 220°C.

10. The process according to at least one of claims 1 to 8, wherein the catalyst hourly space velocity is in the range from 0.05 to 0.6 kg of sugar alcohol per liter of catalyst and hour, and the temperature is in the range from 220 to 350°C.

11. The process according to claim 10, wherein the temperature is in the range from 150 to 220°C.

12. The process according to at least one of claims 1 to 11, wherein the reaction effluent is worked up by distillation.

13. The process according to claim 12, wherein the reaction effluent is dewatered before the distillative workup.

14. The process according to at least one of claims 1 to 13, which is performed in two stages, the sugar alcohols first being reacted with hydrogen in the presence of a catalyst in the first stage and, once a portion of the sugar alcohols has been converted, the aminating agent being added to the reaction mixture in a second stage.

15. The process according to at least one of claims 1 to 14, wherein the aminating agent used is ammonia.

**Revendications**

1. Procédé de fabrication d'amines par mise en réaction de sucres-alcools avec de l'hydrogène et un agent d'amination, choisi dans le groupe constitué par l'ammoniac, la méthylamine, la diméthylamine, l'éthylamine, la diéthylamine, la n-propylamine, la di-n-propylamine, l'iso-propylamine, la di-isopropylamine, l'isopropyléthylamine, la n-butylamine, la di-n-butylamine, la s-butylamine, la di-s-butylamine, l'isobutylamine, la n-pentylamine, la s-pentylamine, l'isopentylamine, la n-hexylamine, la s-hexylamine, l'isohexylamine, la cyclohexylamine, l'aniline, la toluidine, la pipéridine, la morpholine et la pyrrolidine, en présence d'un catalyseur à une température de 100 °C à 400 °C et à une pression de 1 à 40 Mpa, le sucre-alcool étant le sorbitol.

2. Procédé selon la revendication 1, **caractérisé en ce que** le sucre-alcool a été obtenu par hydrogénation du sucre correspondant.

3. Procédé selon au moins l'une quelconque des revendications 1 à 2, **caractérisé en ce que** le catalyseur contient un métal ou plusieurs métaux ou un ou plusieurs composés contenant de l'oxygène des métaux des groupes 8 et/ou 9 et/ou 10 et/ou 11 du tableau périodique des éléments.

4. Procédé selon la revendication 3, **caractérisé en ce que** le catalyseur contient Ni ou NiO et/ou Co ou CoO.

5. Procédé selon la revendication 3, **caractérisé en ce que** le catalyseur est un catalyseur en éponge selon Raney contenant Ni et/ou Co.

6. Procédé selon la revendication 3, **caractérisé en ce que** le catalyseur contient un métal ou plusieurs métaux de la 5$^e$ période des groupes 8 et/ou 9 et/ou 10 et/ou 11 du tableau périodique des éléments.

7. Procédé selon la revendication 3, **caractérisé en ce que** le catalyseur contient Cu ou CuO.

8. Procédé selon la revendication 3, **caractérisé en ce que** le catalyseur contient Ir.

9. Procédé selon au moins l'une quelconque des revendications 1 à 8, **caractérisé en ce que** le chargement du catalyseur se situe dans la plage allant de 0,1 à 1,2 kg de sucre-alcool par litre de catalyseur et par heure, et la température se situe dans la plage allant de 150 à 220 °C.

10. Procédé selon au moins l'une quelconque des revendications 1 à 8, **caractérisé en ce que** le chargement du catalyseur se situe dans la plage allant de 0,05 à 0,6 kg de sucre-alcool par litre de catalyseur et par heure, et la température se situe dans la plage allant de 220 à 350 °C.

11. Procédé selon la revendication 10, **caractérisé en ce que** la température se situe dans la plage allant de 150 à 220 °C.

12. Procédé selon au moins l'une quelconque des revendications 1 à 11, **caractérisé en ce que** la sortie de réaction est traitée par distillation.

13. Procédé selon la revendication 12, **caractérisé en ce que** la sortie de réaction est déshydratée avant le traitement par distillation.

14. Procédé selon au moins l'une quelconque des revendications 1 à 13, **caractérisé en ce que** le procédé est réalisé en deux étapes, la réaction de sucres-alcools avec de l'hydrogène en présence d'un catalyseur ayant tout d'abord lieu lors de la première étape et, après qu'une partie des sucres-alcools ait été transformée, l'agent d'amination étant ajouté au mélange réactionnel lors d'une deuxième étape.

15. Procédé selon au moins l'une quelconque des revendications 1 à 14, **caractérisé en ce que** l'ammoniac est utilisé en tant qu'agent d'amination.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2016962 A
- EP 0255033 A2
- EP 0399488 A2
- EP 1412083 A
- EP 1399255 A
- EP 2007052013 W
- EP 0636409 A
- EP 0742045 A
- EP 696572 A
- EP 963975 A
- DE 2445303 A
- WO 9636589 A
- EP 1106600 A2
- EP 1318128 A2
- FR 2603276 A
- GB 1102370 A
- EP 1312600 A

**In der Beschreibung aufgeführte Nicht-Patentliteratur**

- **H. KELKENBERG.** *Tenside Surfactants Detergents,* 1988, vol. 25, 8-13
- **A. FISCHER ; T. MALLAT ; A. BAIKER.** *Catalysis Today,* 1997, vol. 37, 167-189
- **P. BRANDES ; C. STOEHR.** *J. Prakt. Chem.,* 1896, vol. 54, 481-495
- **C.-K. SHU.** *J. Agric. Food. Chem,* 1998, vol. 46, 1129-1131
- Sugar Alcohols. Ullmann's Encyclopedia of Industrial Chemistry. Wiley-VCH-Verlag, 2007
- **M. S. WAINRIGHT.** Handbook of Heterogeneous Catalysis. Wiley-VCH, 1997, vol. 1, 64 ff
- **A. B. STILES.** Catalyst Manufacture - Laboratory and Commercial Preperations. Marcel Dekker, 1983
- **A. B. STILES.** Catalyst Manufacture. Marcel Dekker, Inc, 1983, 15
- Catalysis and Catalysts. Ullmann's Encyclopedia Electronic Release. 2000, 28-32
- **ERTL ; KNÖZINGER ; WEITKAMP.** Handbook of Heterogenoeous Catalysis. VCH Weinheim, 1997, 98 ff